(19) Europäisches Patentamt
European Patent Office
Office européen des brevets

(11) Publication number: **0 403 251 A2**

# (12) EUROPEAN PATENT APPLICATION

(21) Application number: 90306440.0

(22) Date of filing: 13.06.90

(51) Int. Cl.5: A61K 31/44, A61K 31/00

(30) Priority: 13.06.89 US 365387

(43) Date of publication of application: 19.12.90 Bulletin 90/51

(84) Designated Contracting States: AT BE CH DE DK ES FR GB GR IT LI LU NL SE

(71) Applicant: SMITHKLINE BEECHAM CORPORATION
P.O. Box 7929 1 Franklin Plaza
Philadelphia Pennsylvania 19101(US)

(72) Inventor: Hanna, Nabil
539 Seirra 108
Solana Beach, CA 92075(US)

(74) Representative: Giddings, Peter John, Dr. et al
Smith Kline & French Laboratories Ltd.
Corporate Patents Mundells
Welwyn Garden City Hertfordshire AL7 1EY(GB)

## (54) Monokine activity interference.

(57) A method of treating a human afflicted with a T Cell Viral (TIV) infection, such as a human immunodeficiency virus (HIV) infection ,which comprises administering to such human an effective, monokine activity interfering amount of a monokine activity interfering agent.

EP 0 403 251 A2

# MONOKINE ACTIVITY INTERFERENCE

## BACKGROUND OF THE INVENTION

This invention relates to a method of treating a human afflicted with a human immunodeficiency virus (HIV), which comprises administering to such human an effective amount of a monokine activity interfering agent.

The human acquired immune deficiency syndrome (AIDS) results from the infection of T lymphocytes with Human Immunodeficiency Virus (HIV). At least three types or strains of HIV have been identified, i.e., HIV-1, HIV-2 and HIV-3. As a consequence of HIV infection, T-cell mediated immunity is impaired and infected individuals manifest severe opportunistic infections and/or unusual neoplasms. There is a continuing need for agents which are useful in inhibiting further disease progress in an already infected individual.

Bender et al., U.S. Patent Number 4,794,114, issued December 27, 1988, disclose a method of inhibiting the production of IL-1 by monocytes and/or macrophages in a human in need thereof which comprises administering to such human an effective, interleukin-1 production inhibiting amount of a compound of the formula:

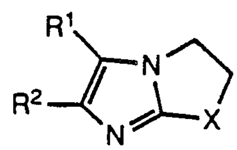

wherein:

One of $R^1$ and $R^2$ must be 4-pyridyl and the other is selected from monohalosubstituted phenyl wherein said substituent is selected from halo or $C_{1-4}$ alkoxy;
X is $CH_2$, $CH_2CH_2$ or S(0)n; and
n is 0, 1 or 2;
or a pharmaceutically acceptable salt thereof.

Bender et al., U.S. Patent Number 4,778,806, issued October 18, 1988, claim a method of inhibiting the production of interleukin-1 by monocytes and/or macrophages in a human in need thereof which comprises administering to such human an effective, interleukin-1 production inhibiting amount of a compound of the formula:

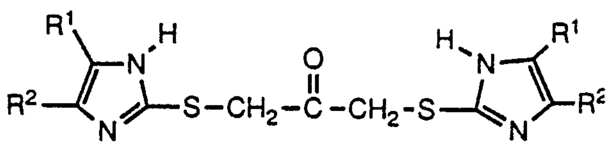

wherein:
one of $R^1$ and $R^2$ is 4-pyridyl and the other is monohalosubstituted phenyl; or a pharmaceutically acceptable salt thereof.

Bender et al., U.S. Patent Number 4,780,470, issued October 25, 1988, claim a method of inhibiting the production of interleukin-1 by monocytes and/or macrophages in a human in need thereof which comprises administering to such human an effective, interleukin-1 production inhibiting amount of a compound of the formula:

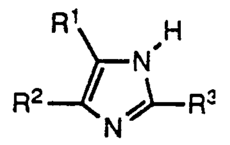

wherein:
One of R' and $R^2$ is 4-pyridyl and the other is selected from monohalosubstituted phenyl; and
$R^3$ is S or $SCF_2CF_2H$;
or a pharmaceutically acceptable salt thereof.

Folks et al., J. Immunol., 136, 40-49 (1986), discuss that cytokine-induced increase of HIV expression in a chronically infected macrophage cell line was associated with the concomitant and selective increase of IL-1 production.

Koyanagi et al., Science, 241, 1673 (1988), discuss that treatment of primary human mononuclear phagocytes with hematopoeitic growth factors including macrophage colony stimulating factor (M-CSF), granulocyte macrophage colony stimulating factor (GM-CSF) and interleukin-3 (IL-3) stimulated HIV production in these cells.

Clouse et al., J. Immunol., 142, 431 (1989), discuss that monokines secreted by activated human monocytes induced elevated levels of HIV expression in a chronically infected human T cell clone. The monokine involved in this process was identified as TNFa.

Gowda et al., J. Immunol., 142, 773 (1989), discuss that T cell activation is required for HIV entry and HIV-dependent cell fusion.

Zagury et al., Science, 231, 850 (1986), discuss that T cell activation is required for HIV gene expression.

Wright et al., J. Immunol., 141, 99 (1988), discuss that monocytes from HIV-infected patients produced large amounts of TNFa and IL-1 upon culturing in vitro.

Ensoli et al., Science, 243, 223 (1989), discuss that IL-1 is produced by cells from Kaposi's sarcoma lesions of HIV-infected patients, and that antibodies to IL-1 inhibited cell proliferation in vitro.

Beutler et al., Nature (London), 316, 552-554 (1985), discuss the role of TNFa in cachexia.

Beutler et al., J. Immunol., 135, 3969-3971 (1985), discuss the role of IL-1 in cachexia.

Baracos et al., N. Eng. J. Med., 308, 553-558 (1983), discuss the role of IL-1 in muscle degeneration.

Chiebowski et al., Nutr. Cancer, 7, 85 (1985), discuss HIV-associated states of cachexia and muscle degradation.

Lahdevirta et al., The American J. Med., 85, 289 (1988), discuss that TNFa is involved in the HIV-associated states of cachexia and muscle degradation.

Wright et al., J. Immunol. 141(1):99-104 (1988) suggests a possible role for TNF in AIDS cachexia by elevated serum TNF and high levels of spontaneous TNF production in peripheral blood monocytes from patients.

Lee et al., Int. J. Immuunopharmac., 10(7), 835 (1988), discuss that [5-(4-pyridyl)-6(4-fluorophenyl)-2,3-dihydroimidazo(2,1-B)thiazole], which inhibits both 5-lipoxygenase and cyclooxygenase-mediated arachidonate metabolism, was shown to be a potent inhibitor of IL-1 production by bacterial lipopolysaccharide (LPS)-stimulated human monocytes.

Badger et al., Circulatory Shock, 27(1), 51-61 (1989), discuss the protection by [5-(4-pyridyl)-6(4-fluorophenyl)-2,3-dihydroimidazo(2, 1-B)thiazole] against two mouse models of LPS-induced shock and inhibition of circulating levels of TNFa.

Folks et al., Proc. Natl. Acad. Sci. USA. 86:2365-2368 (1989) suggests that TNF is implicated in the stimulation of viral replication of latent HIV in T-cell and macrophage lines which can be induced by TNF.

Osborn et al.,Proc. Natl. Acad. Sci. USA 86:2336-2340 (1989) suggests that a molecular mechanism for the virus inducing activity of TNF is due to TNF's ability to activate a gene regulatory protein (NF-kB) found in the cytoplasm of cells, which promotes HIV replication through binding to a viral regulatory gene sequence (LTR)

Yale University, European Patent Application Publication Number 0,230,574 A2, published August 6, 1987, claims a method for producing pharmaceutical compositions for treating patients infected with LAV/HTLV III virus wherein such composition contains a compound which inhibits the production and/or the activity of mononuclear cell derived cytotoxic factors, such as lymphotoxin, tumor necrosis factor, leukoregulin and natural killer cytotoxic factor.

## SUMMARY OF THE INVENTION

This invention relates to a method of treating a human afflicted with a human immunodeficiency virus (HIV), which comprises administering to such human an effective, monokine activity interfering amount of a monokine activity interfering agent.

## DETAILED DESCRIPTION OF THE INVENTION

The human acquired immune deficiency syndrome (AIDS) results from the infection of T lymphocytes with Human Immunodeficiency Virus (HIV). HIV entry into the T lymphocyte requires T lymphocyte activation. Other viruses, such as HIV-1, HIV-2 infect T lymphocytes after T Cell activation and such virus protein expression and/or replication is mediated or maintened by such T cell activation, for example. It has now been discovered that monokines are implicated in the infection of T lymphocytes with HIV by playing a role in maintaining T lymphocyte activation. Furthermore, once an activated T lymphocyte is infected with HIV, the T lymphocyte must continue to be maintained in an activated state to permit HIV gene expression and/or HIV replication. It has now also been discovered that monokines are implicated in activated T-cell mediated HIV protein expression and/or virus replication by playing a role in maintaining T lymphocyte activation. Therefore, interference with monokine activity, such as by inhibition of monokine production, in an HIV-infected individual aids in limiting the maintenance of T cell activation thereby reducing the progression of HIV infectivity to previously uninfected cells which results in a slowing or elimination of the progression of immune dysfunction caused by HIV infection. It has now also been discovered that monokines are implicated in certain disease associated problems such as cachexia and muscle degeneration. Therefore, interference with monokine activity, such as by inhibition of monokine production, in an HIV-infected individual aids in enhancing the quality of life of HIV-infected patients by reducing the severity of monokine-mediated disease associated problems such as cachexia and muscle degeneration. Thus, it is an object of this invention to provide a method for treating an HIV-infected human by administering an effective, monokine activity interfering amount of a monokine activity interfering agent to an HIV-infected human.

By the term "monokine" as used herein is meant any cytokine produced and secreted by a macrophage and/or monocyte of a HIV-infected human provided that such monokine is implicated in (a) the initiation and/or maintenance of T cell activation and/or activated T cell-mediated HIV gene expression and/or replication, and/or (b) any monokine-mediated disease associated problem such as cachexia or muscle degeneration. Examples of such monokines include, but are not limited to, Interleukin-1 (IL-1), Tumor Necrosis Factor-alpha (TNFa) and Tumor Necrosis Factor beta (TNFb).

As TNF-$\beta$ (also known as lymphotoxin) has close structural homology with TNF-a (also known as cachectin) and since each induces similiar biologic responses and binds to the same cellular receptor, both TNF-a and TNF-$\beta$ are both inhibited by the compounds of the present invention and thus TNF-a and TNF-b are herein after referred to as "TNF" unless specifically delineated otherwise.

By the term "monokine activity interfering agent" as used herein is meant any compound which is useful for interfering with the activity of any monokine, such as those compounds which are useful in a method of inhibiting the production of IL-1 or TNF by monocytes and/or macrophages in a human in need thereof as claimed in any of U.S. Patent Number 4,794,114, issued December 27, 1988; U.S. Patent Number 4,788,806, issued October 18, 1988; U.S. Patent Number 4,780,470, U.S. patent application Bender et al., U.S.S.N. 07/365,349, June 13, 1989, and Bender et al., PCT Serial number unknown, filed contemporaneously herewith, Attorney's Docket No. 14446-1, the entire disclosures all of which are hereby incorporated by reference.

By the term "interfering with the activity of any monokine" is meant the down regulation of either the *in vivo* levels of such monokine or the activity of such monokine in a HIV-infected human to levels which interfere with T cell activation and/or activated T cell-mediated HIV gene expression and/or replication to an extent that slows disease progression.

Preferably such monokine activity interfering agent is one which is useful in inhibiting the production of any monokine, such as an agent which inhibits IL-1 production and/or TNFa production, in an HIV-infected patient. By the term "inhibiting the production of any monokine" is meant the lowering of *in vivo* levels of such monokine in a HIV-infected human to levels which interfere with T cell activation and/or activated T cell-mediated HIV gene expression and/or replication to an extent that slows disease progression. By the term "production of any monokine by monocytes and/or macrophages" is meant the *in vivo* release of such monokine by such cells.

Compounds which are useful in the method of the subject invention include those described in Bender et al., U.S. Patent Number 4,794,114, issued December 27, 1988, the entire disclosure of which is hereby incorporated by reference, which discloses a method of inhibiting the production of IL-1 by macrophages and/or monocytes in a human in need thereof by administering an effective amount of a compound of Formula (I):

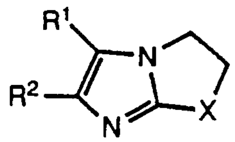

wherein:
One of $R^1$ and $R^2$ must be 4-pyridyl and the other is selected from monohalosubstituted phenyl wherein said substituent is selected from halo or $C_{1-4}$ alkoxy;
X is $CH_2$, $CH_2CH_2$ or S(O)n; and
n is 0, 1 or 2;
or a pharmaceutically acceptable salt there of Compounds described in U.S. 4,794,114 which are preferred for use in the method of the subject invention include those listed in the following Table A.

TABLE A

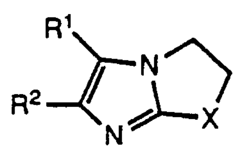

| $R^1$ | $R^2$ | X | n |
|---|---|---|---|
| 4-pyridyl | Fd[a] | $S(O)_n$ | 0 |
| 4-pyridyl | Fd | $S(O)_n$ | 1 |
| 4-pyridyl | Fd | $S(O)_n$ | 2 |
| Fd | 4-pyridyl | $S(O)_n$ | 0 |
| 4-pyridyl | Fd | $CH_2$ | - |
| 4-pyridyl | MeOd[b] | $S(O)_n$ | 0 |
| 4-pyridyl | MeOd | $S(O)_n$ | - |
| 4-pyridyl | MeOd | $S(O)_n$ | - |

[a]Fd = 4-fluorophenyl
[b]MeOd = 4-methoxyphenyl

The most preferred compound of TABLE A for use in the method of the subject invention is the compound known as 5-(4-pyridyl)-6(4-fluorophenyl)-2,3-dihydroimidazo(2,1-B)-thiazole which has been shown to inhibit both IL-1 and TNFa production by monocytes. See, Lee et al., Int. J. Immuunopharmac., 10(7), 835 (1988) and Badger et al., Circulatory Shock, 27(1), 51-61 (1989), the disclosures of both of which are hereby incorporated by reference.

Additional compounds which are useful in the method of the subject invention include those described in Bender et al., U.S. Patent Number 4,778,806, issued October 18, 1988, the entire disclosure of which is hereby incorporated by reference, which discloses a method of inhibiting the production of IL- 1 by monocytes and/or macrophages in a human in need thereof which comprises administering to such human an effective, interleukin- 1 production inhibiting amount of a compound of Formula (II):

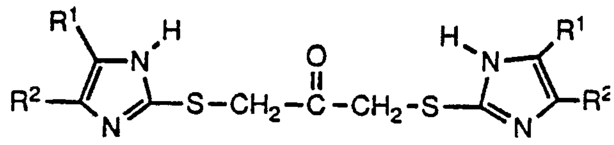

wherein:
one of $R^1$ and $R^2$ is 4-pyridyl and the other is monohalosubstituted phenyl; or a pharmaceutically acceptable salt thereof.

A compound described in U.S. 4,778,806 which is preferred for use in the method of the subject invention is one in which $R^1$ is 4-pyridyl and $R^2$ is 4-fluorophenyl.

Additional compounds which are useful in the method of the subject invention include those described in Bender et al., U.S. Patent Number 4,780,470, issued October 25, 1988, the entire disclosure of which is hereby incorporated by reference, which discloses a method of inhibiting the production of IL-1 by monocytes and/or macrophages in a human in need thereof which comprises administering to such human an effective, interleukin-1 production inhibiting amount of a compound of Formula (III):

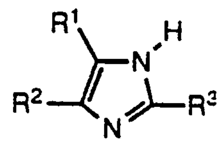

wherein:
One of $R^1$ and $R^2$ is 4-pyridyl and the other is selected from monohalosubstituted phenyl; and
$R^3$ is S or $SCF_2CF_2H$;
or a pharmaceutically acceptable salt thereof.

Compounds described in U.S. 4,780,470 which are preferred for use in the method of the subject invention are the ones in which $R^2$ is 4-pyridyl, $R^1$ is 4-fluorophenyl and $R^3$ is either SH or $SCF_2SF_2H$.

Additional compounds which are useful in the method of the subject invention include those described in U.S. patent application Bender et al., U.S.S.N. 07/365,349 , June 13, 1989, and in Bender et al. PCT Application, number unknown, Attorney's Docket Number SKB 14446-1, filed contemporaneously herewith, the entire disclosures both of which are hereby incorporated by reference, which collectively describe a method of inhibiting the production of IL-1 by monocytes and/or macrophages in a human in need thereof which comprises administering to such human an effective, interleukin-1 production inhibiting amount of a compound of Formula (IV):

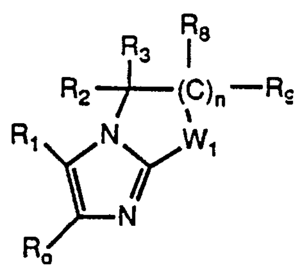

wherein:
$W_1$ is $-(CR_4R_5)-(CR_6R_7)-$, $-CR_5=CR_7-$, $-N=CR_7-$, $-S(O)_m-$ or -O-;
one of $R_1$ and $R_0$ is 4-pyridyl or $C_{1-4}$ alkyl-4-pyridyl, provided that when $R_1$ is $C_{1-4}$ alkyl-4-pyridyl the alkyl substituent is located at the 2-position of the pyridine ring, and the other of $R_1$ and $R_0$ is

(a) phenyl or monosubstituted phenyl wherein said substituent is $C_{1-3}$ alkylthio, $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl, 1-acyloxy-1-alkylthio, $C_{1-2}$ alkoxy, halo, $C_{1-4}$ alkyl or Z wherein Z is - S-S - $Z_1$ and $Z_1$ is phenyl or $C_{1-9}$ alkyl ; or

(b) disubstituted phenyl wherein said substitutents are, independently, $C_{1-3}$ alkylthio, $C_{1-2}$ alkoxy, halo or $C_{1-4}$ alkyl; or

(c) disubstituted phenyl wherein one of said substituents is $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl or 1-acyloxy-1-alkylthio and the other is $C_{1-2}$ alkoxy, halo, or $C_{1-4}$ alkyl;

(d) disubstituted phenyl wherein the substituents are the same and are $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl or 1-acyloxy-1-alkylthio or wherein the substituents together form a methylene dioxy group;or

(e) monosubstituted phenyl wherein the substituent is

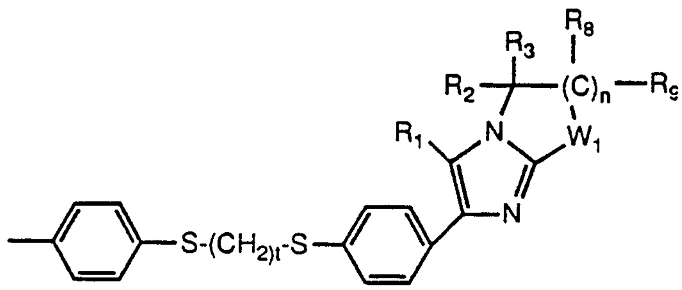

t is 0 or 1; $W_1$, $R_1$, $R_2$, $R_3$, $R_4$, $R_5$, $R_6$, $R_7$, $R_8$ and $R_9$ are as defined above; provided that:

(1.) when $W_1$ is $-(CR_4RS_5)-(CR_6R_7)-$ then

n is 0 or 1; and

$R_2$, $R_3$, $R_4$, $R_5$, $R_6$, $R_7$, $R_8$, and $R_9$ are, independently, -H or $C_{1-2}$ alkyl; and

when $R_1$ or $R_0$ is 4-pyridyl, the other of $R_1$ and $R_0$ is other than mono-$C_{1-2}$ alkoxy-substituted phenyl or mono-halo-substituted phenyl; or

when n is 0, and $R_4$ and $R_5$ together form an oxo; $R_4$ and $R_5$ are both fluoro, or one of $R_4$ and $R_5$ is H and the other OH; or

(2.) when $W_1$ is $-CR_5=CR_7-$ or $-N=CR_7-$ then

n is 1;

$R_3$, $R_5$, $R_7$ and $R_9$ are, independently, -H or $C_{1-2}$ alkyl; and

$R_2$ and $R_8$ together represent a double bond in the B ring such that the B ring is an aromatic pyridine or pyrimidine ring;

(3.) when $W_1$ is $S(O)_m$ then

m is 0, 1 or 2;

n is 1 or 2; and

$R_3$ and $R_9$ are, independently, -H or $C_{1-2}$ alkyl;

$R_2$ and $R_8$ are, independently, -H or $C_{1-2}$ alkyl or $R_2$ and $R_8$ together represent a double bond in the B ring such that the B ring is an aromatic thiazole ring;

further provided that:

(a) when $R_2$ and $R_8$ are, independently, -H or $C_{1-2}$ alkyl and $R_1$ or $R_0$ is 4-pyridyl, then the other of $R_1$ and $R_0$ is other than mono-$C_{1-2}$ alkoxy-substituted phenyl or mono-halo-substituted phenyl; and

(b) when $R_2$ and $R_8$ together represent a double bond in the B ring such that the B ring is an aromatic thiazole ring, then m is 0 and n is 1; and

(4) when $W_1$ is -O- then

n is 1;

$R_3$ and $R_9$ are, independently, -H or $C_{1-2}$ alkyl; and

$R_2$ and $R_8$ together represent a double bond in the B ring such that the B ring is an aromatic oxazole ring; or

the pharmaceutically acceptable salts thereof.

Compounds of the general bicyclic ring structure

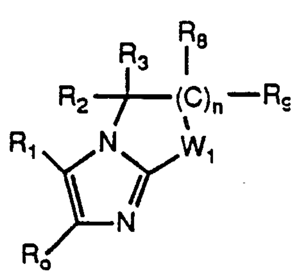

are herein defined to have two rings, the first ring, containing the dihydroimidazole and substiutent groups $R_0$ and $R_1$, is defined as the "A" ring. The saturated or unsaturated ring containing the $W_1$ term and (C)n term, etc. is defined as the "B" ring.

Compounds described in Bender et al., U.S.S.N. 07/365,349 June 13, 1989, and in Bender et al. PCT Serial number unknown, filed contemporaneously herewith, Attorneys Docket Number SKB 14446-1, which are preferred for use in the method of the subject invention include those wherein:

$W_1$ is $-(CR_4R_5)-(CR_6R_7)-$, $-CR_5=CR_7-$, or $-S(O)_m-$;

one of $R_1$ and $R_0$ is 4-pyridyl or $C_{1-2}$ alkyl-4-pyridyl, provided that when $R_1$ is $C_{1-2}$ alkyl-4-pyridyl the alkyl substituent is located at the 2-position of the pyridine ring, and the other of $R_1$ and $R_0$ is

(a) monosubstituted phenyl wherein said substituent is $C_{1-2}$ alkylthio, $C_{1-2}$ alkylsulfinyl, 1-acyloxy-1-alkylthio, $C_{1-2}$ alkoxy or halo, or

(b) disubstituted phenyl wherein said substitutents are, independently, $C_{1-2}$ alkylthio or $C_{1-2}$ alkoxy, or

(c) disubstituted phenyl wherein one of said substituents is $C_{1-2}$ alkylsulfinyl or 1-acyloxy-1-alkylthio and the other is $C_{1-2}$ alkoxy, or

(d) disubstituted phenyl wherein the substituents are the same and are $C_{1-2}$ alkylsulfinyl or 1-acyloxy-1-alkylthio or wherein the substituents together form a methylene dioxy group; provided that:

(1.) when $W_1$ is $-(CR_4R_5)-(CR_6R_7)-$ then

n is 0 or 1; and

$R_2$, $R_3$, $R_4$, $R_5$, $R_6$, $R_7$, $R_8$, and $R_9$ are -H; and

when $R_1$ or $R_0$ is 4-pyridyl, the other of $R_1$ and $R_0$ is other than mono-$C_{1-2}$ alkoxy-substituted phenyl or mono-flouro-substituted phenyl;

(2.) when $W_1$ is $-CR_5=CR_7-$ then

n is 1;

$R_3$, $R_5$, $R_7$ and $R_9$ are -H; and

$R_2$ and $R_8$ together represent a double bond in the B ring such that the B ring is an aromatic pyridine ring;

(3.) when $W_1$ is $S(O)_m$ then

m is 0, 1 or 2;

n is 1 or 2; and

$R_3$ and $R_9$ are -H;

$R_2$ and $R_8$ are -H or $R_2$ and $R_8$ together represent a double bond in the B ring such that the B ring is an aromatic thiazole ring;

further provided that:

(a) when $R_2$ and $R_8$ are -H and $R_1$ or $R_0$ is 4-pyridyl, then the other of $R_1$ and $R_0$ is other than mono-$C_{1-2}$ alkoxy-substituted phenyl or mono-halo-substituted phenyl; and

(b) when $R_2$ and $R_8$ together represent a double bond in the B ring such that the B ring is an aromatic thiazole ring, then m is 0 and n is 1; and

(4) when $W_1$ is -O- then

n is 1;

$R_3$ and $R_9$ are -H; and

$R_2$ and $R_8$ together represent a double bond in the B ring such that the B ring is an aromatic oxazole ring; or a pharmaceutically accetptable salt thereof.

Compounds described in Bender et al., U.S.S.N. 07/365,349, filed June 13, 1989, and in Bender et al., PCT application, number unknown, Attorney's Docket Number SKB 14446-1, filed contemporaneously herewith, which are especially preferred for use in the method of the subject invention include the following:

2-(4-Methylthiophenyl)-3-(4-pyridyl)-6,7-dihydro-[5H]-pyrrolo-[1,2-a]-imidazole;

2-(4-Methylsulfinylphenyl)-3-(4-pyridyl)-6,7-dihydro-[5H]-pyrrolo-[1,2-a]-imidazole;

2-(4-Ethylthiophenyl)-3-(4-pyridyl)-6,7-dihydro-[5H]-pyrrolo-[1,2-a]-imidazole;

2-(4-Ethylsulfinylphenyl)-3-(4-pyridyl)-6,7-dihydro-[5H]-pyrrolo-[1,2-a]-imidazole;

2-(4-Methylthiophenyl)-3-(4-(2-methyl)pyridyl)-6,7-dihydro-[5H]-pyrrolo-[1,2-a]-imidazole;

2-(4-Methylsulfinylphenyl)-3-(4-(2-methyl)pyridyl)-6,7-dihydro-[5H]-pyrrolo-[1,2-a]-imidazole;

2-(4-Methoxyphenyl)-3-(4-pyridyl)-imidazo[1,2-a]-pyridine;

5-(3,4-(M ethylenedioxy)phenyl)-6-(4-pyridyl)-2,3-dihydroimidazo-[2,1-b]-thiazole;

2-(4-Methoxyphenyl)-3-(4-(2-methyl)pyridyl)-6,7-dihydro-[5H]-pyrrolo-[1,2-a]-imidazole;

2-(4-Acetoxymethylthiophenyl)-3-(4-(2-methyl)pyridyl)-6,7-dihydro-[5H]-pyrrolo-[1,2-a]-imidazole;

6-(4-Methylthiophenyl)-5-(4-pyridyl)-2,3-dihydro-imidazo[2,1-b]thiazole;

5-(4-Methylthiophenyl)-6-(4-pyridyl)-2,3-dihydro-imidazo[2,1-b]thiazole;

3-(4-Methylthiophenyl)-2-(4-pyridyl)-6,7-dihydro-[5H]-pyrrolo[1,2-a]imidazole;

2-(4-Propylthiophenyl)-3-(4-pyridyl)-6,7-dihydro-[5H]-pyrrolo-[1,2-a]-imidazole;
2-(4-Methylthiophenyl)-3-(4-(2-ethyl)pyridyl)-6,7-dihydro-[5H]-pyrrolo-[1,2-a]-imidazole; and
2-(4-Mercaptophenyl)-3-(4-pyridyl)-6,7-dihydro-[5H]-pyrrolo-[1,2-a]-imidazole disulfide.

Compounds which are useful in the method of the subject invention for inhibition of TNF include those described in Bender et al. PCT Application, number unknown, Attorneys Docket Number SKB 14446-1, filed contemporaneously herewith, the entire disclosure of which is hereby incorporated by reference, which describes a method of inhibiting the production of TNF by monocytes and/or macrophages in a human in need thereof which comprises administering to such human an effective, TNF production inhibiting amount of a compound of Formula (V):

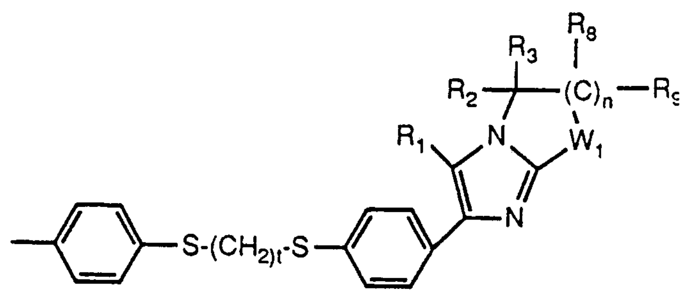

wherein:
$W_1$ is $-(CR_4R_5)-(CR_6R_7)-$, $-CR_5=CR_7-$, $-N=CR_7-$, $-S(O)_m-$ or $-O-$;
one of $R_1$ and $R_0$ is 4-pyridyl or $C_{1-4}$ alkyl-4-pyridyl, provided that when $R_1$ is $C_{1-4}$ alkyl-4-pyridyl the alkyl substituent is located at the 2-position of the pyridine ring, and the other of $R_1$ and $R_0$ is

(a) phenyl or monosubstituted phenyl wherein said substituent is $C_{1-3}$ alkylthio, $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl, 1-acyloxy-1-alkylthio, $C_{1-2}$ alkoxy, halo, $C_{1-4}$ alkyl or Z wherein Z is -S-S- $Z_1$ and $Z_1$ is phenyl or $C_{1-9}$ alkyl; or

(b) disubstituted phenyl wherein said substitutents are, independently, $C_{1-3}$ alkylthio, $C_{1-2}$ alkoxy, halo or $C_{1-4}$ alkyl; or

(c) disubstituted phenyl wherein one of said substituents is $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl or 1-acyloxy-1-alkylthio and the other is $C_{1-2}$ alkoxy, halo, or $C_{1-4}$ alkyl;

(d) disubstituted phenyl wherein the substituents are the same and are $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl or 1-acyloxy-1-alkylthio or wherein the substituents together form a methylene dioxy group;or

(e) monosubstituted phenyl wherein the substituent is

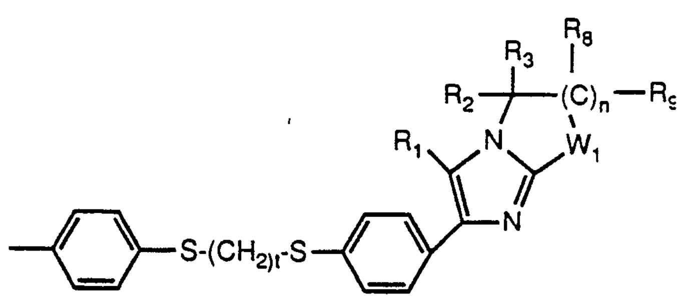

t is 0 or 1; W, $R_1$, $R_2$, $R_3$, $R_4$, $R_5$, $R_6$, $R_7$, $R_8$ and $R_9$ are as defined above; provided that:

(1.) when $W_1$ is $-(CR_4R_5)-(CR_6R_7)-$ then
n is 0; $R_4$ and $R_5$ together may form an oxo; $R_4$ and $R_5$ are both fluoro, or one of $R_4$ and $R_5$ is H and the other OH; or

(2.) when $W_1$ is $-CR_5=CR_7-$ or $-N=CR_7-$ then
n is 1;
$R_3$, $R_5$, $R_7$ and $R_9$ are, independently, -H
or $C_{1-2}$ alkyl; and
$R_2$ and $R_8$ together represent a double bond in the B ring such that the B ring is an aromatic pyridine or pyrimidine ring;

(3.) when $W_1$ is $S(O)_m$ then
m is 0, 1 or 2;
n is 1 or 2; and
$R_3$ and $R_9$ are, independently, -H or $C_{1-2}$ alkyl;
$R_2$ and $R_8$ are, independently, -H or $C_{1-2}$ alkyl or $R_2$ and $R_8$ together represent a double bond in the B ring such that the B ring is an aromatic thiazole ring;
further provided that:

(a) when $R_2$ and $R_8$ are, independently, -H or $C_{1-2}$ alkyl and $R_1$ or $R_0$ is 4-pyridyl, then the other of $R_1$ and $R_0$ mono-flouro-substituted phenyl; and

(b) when $R_2$ and $R_8$ together represent a double bond in the B ring such that the B ring is an aromatic thiazole ring, then m is 0 and n is 1; and

(4) when $W_1$ is -O- then
n is 1;
$R_3$ and $R_9$ are, independently, -H or $C_{1-2}$ alkyl; and
$R_2$ and $R_8$ together represent a double bond in the B ring such that the B ring is an aromatic oxazole ring;
or a pharmaceutically acceptable salt thereof.

Additional compounds which are preferred for use in the method of the subject invention include those wherein:
$W_1$ is $-(CR_4R_5)-(CR_6R_7)-$, $-CR_5=CR_7-$, or $-S(O)_m-$;
one of $R_1$ and $R_0$ is 4-pyridyl or $C_{1-2}$ alkyl-4-pyridyl, provided that when $R_1$ is $C_{1-2}$ alkyl-4-pyridyl the alkyl substituent is located at the 2-position of the pyridine ring, and the other of $R_1$ and $R_0$ is

(a) monosubstituted phenyl wherein said substituent is $C_{1-2}$ alkylthio, $C_{1-2}$ alkylsulfinyl, 1-acyloxy-1-alkylthio, $C_{1-2}$ alkoxy or halo, or

(b) disubstituted phenyl wherein said substitutents are, independently, $C_{1-2}$ alkylthio or $C_{1-2}$ alkoxy, or

(c) disubstituted phenyl wherein one of said substituents is $C_{1-2}$ alkylsulfinyl or 1-acyloxy-1-alkylthio and the other is $C_{1-2}$ alkoxy, or

(d) disubstituted phenyl wherein the substituents are the same and are $C_{1-2}$ alkylsulfinyl or 1-acyloxy-1-alkylthio or wherein the substituents together form a methylene dioxy group; provided that:

(1.) when $W_1$ is $-CR_5=CR_7-$ then
n is 1;
$R_3$, $R_5$, $R_7$ and $R_9$ are -H; and
$R_2$ and $R_8$ together represent a double bond in the B ring such that the B ring is an aromatic pyridine ring;

(2.) when $W_1$ is $S(O)_m$ then
m is 0, 1 or 2;
n is 1 or 2; and
$R_3$ and $R_9$ are -H;
$R_2$ and $R_8$ are -H or $R_2$ and $R_8$ together represent a double bond in the B ring such that the B ring is an aromatic thiazole ring;
further provided that:

(a) when $R_2$ and $R_8$ are -H and $R_1$ or $R_0$ is 4-pyridyl, then the other of $R_1$ and $R_0$ is other than mono-flouro-substituted phenyl; and

(b) when $R_2$ and $R_8$ together represent a double bond in the B ring such that the B ring is an aromatic thiazole ring, then m is 0 and n is 1; and

(4) when $W_1$ is -O- then
n is 1;
$R_3$ and $R_9$ are -H; and
$R_2$ and $R_8$ together represent a double bond in the B ring such that the B ring is an aromatic oxazole ring.

Additional compounds which are useful in the method of the subject invention because they are inhibitors of TNF and IL-1 include 2-(4-Methoxyphenyl)-3- (4-pyridyl)-7-oxo-5,6-dihydro-[7H]-pyrrolo-[1,2-a]-imidazole; 5,6-dihydro-2-(4-Methoxyphenyl)-3-(4-pyridyl)-[7H]-pyrrolo-[1,2-a]-imidazole-7-ol.; and 5,6-dihydro-7,7-difluoro-2-(4-Methoxyphenyl)-3-(4pyridyl)-[7H]-pyrrolo-[1,2-a]-imidazole are described in Tetrahedron Letter, Vol. 30, No. 48, pp. 6599-6602 (1989) the entire disclosure of which is hereby incorporated by reference.

Especially preferred compounds for use in the method of the subject invention are
2-Phenyl-3-pyridyl-6,7-dihydro-[5H]-pyrrolo-[1,2-a]imidazole;
2-4-Bromophenyl-3-pyridyl-6,7-dihydro-[5H]-pyrrolo-[1,2-a]-imidazole;
2-(4-Pyridyl)-3-(4-flurophenyl)6,7-dihydro-[5H]-pyrrolo-[1,2-a] imidazole;

2-(4-Fluorophenyl)-3-(4-pyridyl)-6,7-dihydro-[5H]-pyrrolo[1,2-a]-imidazole;
2-(4-Methylthiophenyl)-3-(4-pyridyl)-6,7-dihydro-[5H]-pyrrolo[1,2-a]-imidazole;
2-(4-Pyridyl)3-(4-methylthiophenyl)-6,7-dihydro-[5H]-pyrrolo[1,2-a]-imidazole;
2-(4-Methylthiophenyl)-3-[4-(2-methylpyridyl)]-6,7-dihydro-[5H]-pyrrolo[1,2-a]-imidazole;
2-(4-Methoxyphenyl)-3-(4-pyridyl)-7-oxo-5,6-dihydro-7H-pyrrolo[1,2-a]imidazole;
5,6-Dihydro-2-(4-methoxyphenyl)-3-(4-pyridinyl)-[7H]-pyrrolo[1,2-a]imidazole-7-ol;
2-(4-Acetoxymethylthiophenyl)-3-(4-pyridyl)-6,7-dihydro[5H]pyrrolo-[1,2-a]-imidazole;
2-(4-Methoxyphenyl)-3-(4-pyridyl)-6,7-dihydro-[5H]-pyrrolo[1,2-a]imidazole;
6-(4-Fluorophenyl)-5-(4′-pyridyl)-2,3-dihydroimidazo[2,1-b]thiazole;
6-(4-Fluorophenyl)-5-(4′-pyridyl)-2,3-dihydroimidazo-[2,1-b]thiazole-1-oxide;
6-(4-Fluorophenyl)-5-(4-pyridyl)-2,3-dihydroimidazo[2,1-b]thiazole-1,1-dioxide;
5-(4-Fluorophenyl)-6-(4′-pyridyl)-2,3-dihydroimidazo[2,1-b]thiazole;
2-(4-Methylsulfoxyphenyl)3-(4-pyridyl)-6,7-dihydro[5H]-pyrrolo[1,2-a]imidazole;
2-(4-Ethylthiophenyl)-3-(4-pyridyl)-6,7-dihydro[5H]-pyrrolo-[1,2-a]imidazole; or
2-(4-Ethylsulfonylphenyl)-3-(4-pyridyl)-6,7-dihydro-[5H]-pyrrolo-[1,2-a]imidazole.

This invention relates to a method of treating a human afflicted with a human immunodeficiency virus, which comprises administering to such human an effective, monokine activity interfering amount of a monokine activity interfering agent. The monokine activity interfering agent is administered to a HIV-infected human in an amount sufficient to interfere with the production or activity of any monokine (a) implicated in T lymphocyte activation to levels which interfere with T cell activation and/or activated T cell-mediated HIV gene expression and/or replication to an extent that slows disease progression and/or (b) implicated in monokine-mediated disease associated problems such as cachexia and muscle degeneration to levels which improve the quality of the life of the HIV-infected individual. Preferably such monokine activity interfering agent is one which inhibits IL-1 production, TNFa or TNFb production by monocytes and/or macrophages in an HIV-infected patient. In an HIV-infected human manifesting immune dysfunction, treatment with an effective amount of a monokine activity interfering agent will initially result in a slowing of the rate of T cell depletion, thereby slowing disease progression. It is expected that T cell depletion will gradually cease and that T cell counts and T4/T8 ratios will begin to normalize. In an HIV-infected human not yet manifesting immune dysfunction, treatment with an effective amount of a monokine activity interfering agent will result in a slowing of the rate of T cell depletion, thereby slowing disease progression and delaying immune dysfunction manifestation. In an HIV-infected human manifesting monokine-mediated disease associated problems such as cachexia or muscle degeneration, treatment with an effective amount of a monokine activity interfering agent will initially result in a slowing of the rate of the progression of the disease associated problem, thereby slowing disease progression. It is expected that the progression of the disease associated problem will eventually cease and reverse, thereby enhancing the quality of life of the HIV-infected individual treated in such a manner.

It will be recognized by one of skill in the art that the actual amount of a monokine activity interfering agent required for therapeutic effect will, of course, vary with the agent chosen, the route of administration desired, the nature and severity of the HIV-infection and the particular condition of the HIV-infected human undergoing treatment, and is ultimately at the discretion of the physician. It will also be recognized by one of skill in the art that the optimal quantity and spacing of individual dosages of a monokine activity interfering agent will be determined by the nature and extent of the condition being treated, the form, route and site of administration, and the particular patient being treated, and that such optimums can be determined by conventional techniques. It will also be appreciated by one of skill in the art that the optimal course of treatment, i.e., the number of doses of a monokine activity interfering agent given per day for a defined number of days, can be ascertained by those skilled in the art using conventional course of treatment determination tests.

The monokine activity interfering agent is administered orally, topically, parenterally or by inhalation in conventional dosage forms prepared by combining such agent with standard pharmaceutical carriers according to conventional procedures in an amount sufficient to produce therapeutic monokine activity interfering activity.

The pharmaceutical carrier employed can be readily determined by one of skill in the art who will recognize that such determination will depend upon various well-known factors such as the nature, quantity and character of the particular monokine activity interfering agent being employed and the form and route of administration desired.

The method of the subject invention may be carried out by delivering the monokine activity interfering agent parenterally, orally, topically or by inhalation depending upon various factors such as the nature of the agent and the desired site of inhibition.

In general, an initial treatment regimen can be copied from that known to be effective in interfering with monokine activity for the particular monokine activity interfering agent employed. Treated individuals will be regularly checked for T cell numbers and T4/T8 ratios and/or for progression of monokine-mediated disease associated problems such as cachexia or muscle degeneration. If no effect is seen following the normal treatment regimen, then the amount of the monokine activity interfering agent administered is increased, e.g., by fifty percent per week.

As stated above, the method of the subject invention may be carried out by delivering the monokine activity interfering agent topically. By topical administration is meant non-systemic administration and includes the application of a monokine activity interfering agent externally to the epidermis, to the buccal cavity and instillation of such a compound into the ear, eye and nose, and where the compound does not significantly enter the blood stream.

A suitable monokine activity interfering dose of any IL-1 production inhibiting compound disclosed in any of the method of use claims of U.S. Patent Numbers 4,794,114, 4,778,806 and 4,780,470, U.S. patent application Bender et al., U.S.S.N. 07/365,349, June 13, 1989, and Bender et al., PCT Application, number unknown, Attorney's Docket Number SKB 14446-1, filed contemporaneously herewith, is 15 mg to 500 mg of base for topical administration, the most preferred dosage being 1 mg to 100 mg, for example, 5 to 25 mg administered two or three times daily. The daily topical dosage regimen will preferably be from about 2 mg to about 10 mg per site of administration.

A suitable monokine activity interfering dose of any TNF production inhibiting compound is from about 1 mg to about 1000 mg of base for topical administration, the most preferred daily dosage being 15 mg to 500 mg, the single dosage range being about 5mg to 160 mg. The daily topical dosage regimen will preferably be from about 2 mg to about 10 mg per site of administration.

As stated above, the method of the subject invention may be carried out by delivering the monokine activity interfering agent by inhalation. By "inhalation" is meant intranasal and oral inhalation administration. Appropriate dosage forms for such administration, such as an aerosol formulation or a metered dose inhaler, may be prepared by conventional techniques. A suitable monokine activity interfering dose herewith, administered by inhalation is from about 1 mg to about 100 mg per day. More detailed information about inhalation formulation of such compounds is outlined in the specifications for any of the IL-1 production inhibiting compounds disclosed in the method of use claims of U.S. Patent Numbers 4,794,114, 4,778,806 and 4,780,470, U.S. patent application Bender et al., U.S.S.N. 07/365,349 , filed June 13, 1989, and Bender et al., PCT Application, number unknown, Attorney's Docket Number SKB 14446-1, filed contemporaneously herewith..

A suitable monokine activity interfering dose of any TNF production inhibiting compound administered by inhalation is from about 1 mg to about 1000 mg per day. More preferably from about 10mg to about 100mg per day.

As stated above, the method of the subject invention may be carried out by delivering the monokine activity interfering agent parenterally. The term 'parenteral' as used herein includes intravenous, intramuscular, subcutaneous intranasal, intrarectal, intravaginal or intraperitoneal administration. The subcutaneous and intramuscular forms of parenteral administration are generally preferred. Appropriate dosage forms for such administration may be prepared by conventional techniques. A suitable monokine activity interfering dose, and more detailed information about parenteral formulation of such compounds is outlined in the specifications of any IL-1 production inhibiting compound disclosed any of the method of use claims of in U.S. Patent Numbers 4,794,114, 4,778,806 and 4,780,470, U.S. patent application Bender et al., U.S.S.N. 07/365,349, filed June 13, 1989 , and in Bender et al., PCT serial number unknown, Attorney's Docket Number SKB 14446 1, filed contemporaneously herewith. The dose administered parenterally will preferably be from about 1 to about 100 mg per kilogram (kg) of total body weight, most preferably from about 3 to about 60 mg/kg per day.

A suitable monokine activity interfering dose of any TNF production inhibiting compound administered parenterally will preferably be from about 1 to about 100 mg per kilogram (kg) of total body weight, most preferably from about 5 to about 80 mg/kg per day.

As stated above, the method of the subject invention may be carried out by delivering the monokine activity interfering agent orally. Appropriate dosage forms for such administration may be prepared by conventional techniques. A suitable monokine activity interfering dose of any IL-1 production inhibiting compound disclosed in any of the method of use claims of U.S. Patent Numbers 4,794,114, 4,778,806 and 4,780,470, U.S. patent application Bender et al., U.S.S.N. 07/365,349, June 13, 1989, and in Bender et al., PCT Application Number unknown, Attorney's Docket Number SKB 14446-1, filed contemperanously herewith, administered orally will preferably be from about 5 to about 100 mg/kilogram of total body weight per day.

As stated above, the method of the subject invention may be carried out by delivering the monokine activity interfering agent orally. Appropriate dosage forms for such administration may be prepared by conventional techniques. A suitable monokine activity interfering dose of any TNF production inhibiting compound will preferably be from about 1 to about 100 mg per kilogram (kg) of total body weight, most preferably from about 5 to about 80 mg/kg per day. More detailed information about topical, oral, inhalation and parenteral dosage formulations for the TNF inhibiting compounds of the subject application is outlined in the specifications of U.S. patent application Bender et al., U.S.S.N. 07/365,349, filed June 13, 1989 and Bender et al., PCT Application number unknown, Attorney's Docker Number SKB SKB 14446-1, filed contemporaneously herewith.

This invention also relates to a method of treating HIV infection in a human infected with HIV which comprises administering to such human an effective amount of 5-(4-pyridyl)-6-(4-fluorophenyl)-2,3-dihydroimidazo-(2,1-b)-thiazole. By the term "5-(4-pyridyl)-6-(4-fluorophenyl)-2,3-dihydroimidazo-(2, 1-b)-thiazole" as used herein is meant both the base form of the compound as well as all pharmaceutically acceptable salts thereof.

This invention also relates to a method of treating HIV infection in a human infected with HIV which comprises administering to such human an effective amount of 2-(4-methylthiophenyl)-3-(4-pyridyl)-6,7-dihydro-[5H]-pyrrolo[1,2-a]imidazole as well as all pharmaceutically acceptable salts thereof.

A method for the preparation of 5-(4-pyridyl)-6(4-fluorophenyl)-2,3-dihydroimidazo-(2,1-b)-thiazole (including all pharmaceutically acceptable salt forms thereof) is described by U.S. Patent 4,175,127, issued November 20, 1979, and an improved method for its preparation is described by U.S. Patent 4,803,279, issued February 9, 1989, the entire disclosures of both of which patents are hereby incorporated by reference. A method for the preparation of 2-(4-methylthiophenyl)-3-(4-pyridyl)-6,7-dihydro-[5H]-pyrrolo[1,2-a]-imidazole, 4-alkylthiophenyl and 4-alkylksulfinylphenyl derivatives is specifically described in US Patent No. 4,719,218 issued January 12, 1988 and is hereby incorporated by reference.

The compound 5-(4-pyridyl)-6(4-fluorophenyl)-2,3-dihydroimidazo-(2,1-B)-thiazole (hereinafter referred to as "Compound 1") and 2-(4-methylthiophenyl)-3-(4-pyridyl)-6,7-dihydro-[5H]-pyrrolo[1,2-a]-imidazole, (hereinafter referred to as "Compound 2") are administered to a HIV-infected human in an amount sufficient to have a therapeutic effect on such individual's infection by enhancing the quality of life of the infected individual by slowing the progression of the disease and/or by ameliorating HIV associated conditions such as cachexia and muscle degeneration It will be recognized by one of skill in the art that the actual amount of Compounds 1 or 2 as well as other pyrrolo-[2,1-a]-imidazoles and imidazo-[2,1-b]-thiazoles of the compounds described herein required for therapeutic effect will, of course, vary with the route of administration desired, the nature and severity of the HIV-infection and the particular condition of the HIV-infected human undergoing treatment, and is ultimately at the discretion of the physician. It will also be recognized by one of skill in the art that the optimal quantity and spacing of individual dosages of Compounds 1 or 2 will be determined by the nature and extent of the condition being treated, the form, route and site of administration, and the particular patient being treated, and that such optimums can be determined by conventional techniques. It will also be appreciated by one of skill in the art that the optimal course of treatment, i.e., the number of doses of Compounds 1 or 2 given per day for a defined number of days, can be ascertained by those skilled in the art using conventional course of treatment determination tests.

Compounds 1or 2 are administered orally, topically, parenterally or by inhalation in conventional dosage forms prepared by combining such agent with standard pharmaceutical carriers according to conventional procedures.

The pharmaceutical carrier employed can be readily determined by one of skill in the art who will recognize that such determination will depend upon various well-known factors such as the form and route of administration desired.

As stated above, the method of the subject invention may be carried out by delivering Compounds 1 or 2 topically. By topical administration is meant non-systemic administration and includes the application of Compounds 1or 2 externally to the epidermis, to the buccal cavity and instillation of such a compound into the ear, eye and nose, and where the compound does not significantly enter the blood stream. A suitable dose of Compounds 1 or 2 is 1.5 mg to 100 mg of base for topical administration, the most preferred dosage being 1 mg to 500 mg, for example 5 to 25 mg administered two or three times daily. The daily topical dosage regimen will preferably be from about 2 mg to about 10 mg per site of administration. More detailed information about topical formulation of Compound 1 is outlined in the specification of U.S. Patent Number 4,794,114.

As stated above, the method of the subject invention may be carried out by delivering Compounds 1 or 2 by inhalation. By "inhalation" is meant intranasal and oral inhalation admini-stration. Appropriate dosage

forms for such administration, such as an aerosol formulation or a metered dose inhaler, may be prepared by conventional techniques. A suitable dose of Compounds 1 or 2 administered by inhalation is from about 1 mg/kg to about 100 mg/kg per day. More detailed information about inhalation formulation of Compound 1 is outlined in the specification of U.S. Patent Numbers 4,794,114.

As stated above, the method of the subject invention may also be carried out by delivering Compounds 1 or 2 parenterally. The term 'parenteral' as used herein includes intravenous, intramuscular, subcutaneous intranasal, intrarectal, intravaginal or intraperitoneal administration. The subcutaneous and intramuscular forms of parenteral administration are generally preferred. Appropriate dosage forms for such administration may be prepared by conventional techniques. A suitable dose of Compounds 1 or 2 administered parenterally will preferably be from about 1 to about 100 mg per kilogram (kg) of total body weight, most preferably from about 2 to about 60 mg/kg per day. More detailed information about parenteral formulation of Compound 1 is outlined in the specification of U.S. Patent Number 4,794,114.

As stated above, the method of the subject invention may also be carried out by delivering Compounds 1 or 2 orally. Appropriate dosage forms for such administration may be prepared by conventional techniques. A suitable dose of Compounds 1 or 2 administered orally will preferably be from about 1 to about 100 mg/kilogram of total body weight per day. More preferably from about 2mg/kg to about 60mg/kg per day. More detailed information about oral formulation of Compound 1 is outlined in the specification of U.S. Patent Numbers 4,794,114.

This invention relates to the use of a compound of Formula (I), Formula (II), Formula (III), Formula (IV), or Formula (V) or a pharmaceutically acceptable salt thereof in the manufacture of a medicament for the treatment of a human afflicted with a human immunodeficiency virus (HIV), which comprises administering to such human an effective amount of a monokine activity interfering agent of Formula's (I) -(V).

## UTILITY EXAMPLES

### EXAMPLE A

Two models of endotoxin shock have been utilized to determine in vivo TNF activity and are described below. The actual protocoll used in the models is described in Utility Model Examples A and B set out below, i.e. P. Acnes model and LPS-GAL model. In these models protection from the lethal effects of endotoxin shock is provided by the compound 6-(4′-Flurophenyl)-5-(4′pyridyl)-2,3-dihydroimidazo-[2,1-b]-thiazole, (herein called Compound 1) and 2-(4-methyl-sulfoxyphenyl)-3-(4-pyridyl)-6,7-dihydro[5H]-pyrrolo-[1,2-a]-imidazole (herein called Compound 2). The data in Figures 1-5 clearly demonstrates the ability of Compounds 1 and 2 to reduce the in vivo level of tumor necrosis factor (TNF).

Compound 2 shows a reduction in serum TNF levels in the P acnes/LPS treated mice model as depicted by the data shown in Figure 1, which demonstrates decreased levels of in vivo TNF relative to increased oral dosage of Compound 2. Figure 2 demonstrates inhibition of TNF production, also in the P. acnes/LPS Model for both Compounds 1 and 2.

Figure 3 demonstrates that (intra-peritoneal) i.p.injection of Compound 1 on inhibitis TNF production in the LPS-Gal mouse model of endotoxic shock. Figure 4 shows a comparison of the reduction of serum TNF levels at 100 mg/kg for both Compounds 1 and 2 in the LPS-GAL Model. Figure 5 demonstrates 100% survival rate of the animals with endotoxic shock in the LPS-GAL model after treatment with Compounds 1 and 2 compared to only a 30% survival rate of the animals in the control group.

It has also been determined, using one or both of the in vivo assays described herein, that 2-(4-Ethylsulfinylphenyl)-3-(4-pyridyl)-6,7-dihydro-[5H]-pyrrolo-[1,2-a]-imidazole, 2-(4-methylthiolphenyl)-3-[4-(2-methylpyridyl)]-6,7-dihydro-[5H]-pyrrolo-[1,2-a]-imidazole, and 6-(4-Fluorophenyl)-5-(4-pyridyl)-2,3-dihydroimidazo[2,1-b]thiazole-1,1-dioxide also inhibited in vivo TNF levels as well as protected the animals from endotoxic induced shock.

The data shown herein demonstrate that the compounds of the present invention inhibit TNF production in a mammal. Therefore, the compounds of the present invention are useful as monokine activity interfering agents, e.g. they are useful in inhibiting the production of tumor necrosis factor (TNF) by monocytes or macrophages in a human.

## UTILITY EXAMPLE MODEL A

### Endotoxin Shock in D-gal-Sensitized Mice

The protocol used to test the compound of the method of the subject invention was essentially as has been described in Galanos et al., Proc. Nat'l Acad. Sci USA, 76:5939-43 (1979) whose disclosure is herein incorporated by reference. Briefly, D-gal (D(+) Galactosidase) sensitizes various strains of mice to the lethal effects of endotoxin. The administration of D-gal (300-500mg/kg) intra-venously (i.v.) sensitizes the mice to doses of Lipopolysaccharide(LPS ) as low as 0.1 mg. Briefly, male C57BL/6 mice, obtained from Charles River Laboratories (Stone Ridge, New York, USA) of 6-12 weeks of age were injected i.v. with 0.1 mg of LPS from Salmonella typhosa (Difco Laboratories, Detroit, Michigan, USA) admixed with D(+)-gal (Sigma; 500 mg/kg) in 0.20-0.25 ml pyrogen-free saline. Compounds to be tested were administered at various times prior to or following the iv. injection of LPS/D-gal. In this model, the control animals usually die 5-6 hr. following the injection of LPS, although on occasion deaths are seen between 24 and 48 hr.

## UTILITY MODEL EXAMPLE B

### Endotoxin Shock in P. acnes-Sensitized Mice

This model is a modification of the previously described protocol for the in vivo induction of TNF as described in Haranaka et al., Cancer Immunol Immunother, 18:87-90, (1984). Treatment with Proprionibacterium Acnes (P. acnes) (1mg/animal i.p.) renders mice susceptible to the lethal effects of LPS injected 10 days later.

P. acnes was purchased from Burroughs Wellcome (Triangle Park, North Carolina, USA), and 1 microgram (ug)of the heat-killed bacteria was administered in 0.5 ml pyrogen-free saline by intraperitoneal (i.p.) injection to male C57BL/6 mice, obtained from Charles River Laboratories (Stone Ridge, New York, USA) of 6-12 weeks of age. Ten days later, the mice were injected iv. with 1 mg LPS in 0.25 ml saline. Compounds to be tested were administered at various times prior to or following the injection of LPS. The survival of animals was monitored for 1 week.

### Measurement of TNF Activity

Plasma levels of TNF were measured using a modification of the basic sandwich ELISA method described in Winston et al., Current Protocols in Molecular Biology, Pg. 11.2.1, Ausubel et al., Ed. (1987) John Wiley and Sons, New York, USA. The Elisa employed a hampster monoclonal anti-mouse TNF (Genzyme, Boston, MA, USA) as the capture antibody and a polyclonal rabbit anti-murine TNF (Genzyme, Boston, MA, USA) as the detecting antibody. TNF levels in rat samples were calculated from a standard curve genereated with recombinant murine TNF (Genzyme, Boston, MA, USA). TNF levels determined by ELISA correlated with levels detected by the L929 bioassay of Ruff et. al., J. Immunol. 125:1671-1677 (1980), with 1 Unit of activity in the bioassay corresponding to 70 picograms (pg) of TNF in the ELISA. The ELISA detected levels of TNF down to 25 pg/ml.

## UTILITY EXAMPLES

In the following table of the Utility Examples, the following abbreviations are employed:

| ABBREVIATION | FORMULA (I) COMPOUNDS |
|---|---|
| Compound 1 | 6-(4-Flurophenyl)-5-(4′pyridyl)-2,3-dihydroimidazo[2,1-b]-thiazole; |
| Compound 2 | 2-(4-Methylsulfoxyphenyl)3-(4-pyridyl)-6,7-dihydro[5H]-pyrrolo-[1,2-a]imidazole; |
| Compound 3 | 2-(4-methylthiophenyl)-3-(4-pyridyl)-6,7-dihydro-[5H]-pyrrolo[1,2-a]-imidazole; |
| Compound 4 | 2-phenyl-3-pyridyl-6,7-dihydro-[5H]-pyrrolo[1,2-a]imidazole; |
| Compound 5 | 2-p-bromophenyl-3-pyridyl-6,7-dihydro-[5H]-pyrrolo-[1,2-a]-imidazole; |
| Compound 6 | 3-(4-flurophenyl)-2-(4-pyridyl)-6,7-dihydro-[5H]-pyrrolo-[1,2-a]imidazole; |
| Compound 7 | 2-(4-Methoxyphenyl)-3-(4-pyridyl-7-oxo-5,6-dihydro-7-H-pyrrolo-[1,2-a]imidazole; |
| Compound 8 | 5,6-dihydro-2-(4-methoxyphenyl)-3-(4-pyridinyl)-[7H]-pyrrolo[1,2-a]-imidazole-7-ol; |
| Compound 9 | 2-(4-Acetoxymethylthiophenyl)3-(4-pyridyl)-6,7-dihydro[5H]pyrrolo-[1,2-a]-imidazole; |
| Compound 10 | 2-(4-Methoxyphenyl)-3-(4-pyridyl)-6,7-dihydro-[5H]-pyrrolo-[1,2-a]imidazole; |
| Compound 11 | 2-(4-Fluorophenyl)-3-(4-pyridyl-6,7-dihydro-[5H]-pyrrolo[1,2-a]-imidazole; |
| Compound 12 | 6-(4-Fluorophenyl)-5-(4′-pyridyl-2,3-dihydroimidazo[2,1-b]thiazole-1-oxide; |
| Compound 13 | 5-(4-Fluorophenyl)-6-(4′-pyridyl)-2,3-dihydroimidazo[2,1-b]thiazole; |
| Compound 14 | 6-(4-Fluorophenyl)-5-(4-pyridyl)-2,3-dihydroimidazo[2,1-b]thiazole-1,1-dioxide; |
| Compound 15 | 2-(4-Ethylsulfinylphenyl)-3-(4-pyridyl)-6,7-dihydro[5H]pyrrolo[1,2-a]imidazole; |
| Compound 16 | 2-(4-Methylthiophenyl)-3-[4-(2-methylpyridyl)]-6,7-dihydro[5H]pyrrolo-[1,2-a]imidazole. |

## EXAMPLE A

Inhibitory Effect of Compounds on in vitro TNF Production by Human Monocytes

The effects of the compounds enumerated above on the in vitro production of TNF by human monocytes was examined using the following protocol..

Bacterial lipopolysaccharide (LPS) was used to induce TNF production by human peripheral blood monocytes. TNF activity was measured by a modification of Winston et al., ELISA, described below. Human peripheral blood monocytes were isolated and purified from either fresh blood preparations from volunteer donors, from blood bank buffy coats, or from plateletpheresis residues according to the procedure of Colotta et al., J. Immunol., 132, 936 (1984). 1 X $10^6$ of such monocytes were plated in 24-well plates at a concentration of 1-2 million/ml per well. The cells were allowed to adhere for 2 hours, after which time non-adherent cells were removed by gentle washing. Test compounds were then added to the cells for 1 hour (hr) before the addition of LPS (50 ng/ml), and the cultures were incubated at 37°C for an additional 24 hours. At the end of the incubation period, culture supernatants were removed and clarified of cells and all debris. Culture supernatants were immediately assayed for TNF levels in the manner described below.

### MEASUREMENT OF HUMAN TNF:

Levels of TNF were mesured using a modification of the basic sandwich ELISA assay method described in Winston et al., Current Protocols in Molecular Biology. Page 11.2.1, Ausubel et al., Ed. (1987) John Wiley and Sons, New York, USA The Elisa employes a murine monoclonal anti-human TNF antibody, described below, as the capture antibody and a polyclonal rabbit anti-human TNF , described below, as the second antibody. For detection, a peroxidase-conjgated goat anti-rabbit antibody (Boehringer Mannheim, Indianopolis, Indiana, USA, Catalog #605222) was added followed by a substrate for peroxidase (1mg/ml orthophenylenediamine with 0.1% urea peroxide). TNF levels in samples were calculated from a standard curve genereated with recombinant human TNF produced in E. Coli (obtained from SmithKline Beecham Pharmaceuticals, King of Prussisa, PA, USA).

### PRODUCTION OF ANTI-HUMAN TNF ANTIBODIES:

Monoclonal antibodies to human TNF were prepared from spleens of BALB/c mice immunized with

recombinant human TNF using a modification of the method of Kohler and Millstein, Nature 256: 495(1975), the entire disclosure of which is hereby incorporated by reference. Polyclonal rabbit anti-human TNF antibodies were prepared by repeated immunization of New Zeland White (NZW) rabbits with recombinant human TNF emulsified in complete Freund's adjuvant (DIFCO, IL., USA).

The results indicated that human peripheral blood monocytes are exquisitely sensitive to bacterial endotoxin. Nanogram or even picogram quantities of LPS stimulated high levels of TNF production as well as for IL-1 production.

The results of the effects of compounds on the in vitro TNF production by human monocytes are reported in Table B. The compounds wherein the phenyl substituent group of the pyrrolo[2,1-b] imidazoles and the dihydroimidazo [2,1-a] thiazoles of the present invention are not a sulfinyl derivatives, are potent inhibitors of in vitro TNF production by human monocytes. The compounds wherein the substiiuent is a sulfinyl derivatives are not active in the in vitro assay. However, the sulfinyl derivatives function as prodrugs to their corresponding sulfide, i.e., they function in vivo to inhibit the production of TNF, because they are metabolized so that they are reductively converted, in vivo, to their corresponding biologically active alkylthio or alkenylthio form. Proof of this in vivo conversion of the sulfinyl derivatives to a biologically active form is indicated by the in vivo activity of the sulfinyl compounds in the in vivo assay described in Utility Models Examples A and B for Compound 2. The conversion of the sulfinyl/sulfoxy pro-drug derivatives to their active sulfinyl strucutre also holds true for IL-1 activity and is described in detail in U.S. patent application Bender et al., U.S.S.N. 07/365,349, filed June 13, 1989, and in Bender et al., PCT Application, number unknown, Attorneys Docket Number SKB 14446-1, filed contemporaneously herewith,

The results of the effects of compounds of Formula (I) on the in vitro TNF production by human monocytes are reported in Table B.

TABLE B

| LPS induced TNF Human Monocyte data | |
|---|---|
| Compound No. | $IC_{50}$ (mM) |
| 1 | 0.5 |
| 2 (Sulfoxy derivative) | NA |
| 3 (Sulfinyl derivative) | 1.0 |
| 4 | 0.8 |
| 5 | 1.0 |
| 6 | 1.0 |
| 7 | 1.0 |
| 8 | 1.0 |
| 9 | 3.0 |
| 10 | 0.5 |
| 11 | 0.2 |
| 12 | 3.0 |
| 13 | 0.5 |
| 14 | 9.0 |
| 15 | 7.0 |
| 16 | 7.0 |
| NA - not active | |

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore the Examples herein are to be construed as merely illustrative and not a limitation of the scope of the present invention in any way.

## Claims

1. The use of a monokine activity interfering agent in the manufacture of a medicament for treating a human afflicted with a human immunodeficiency virus (HIV).

2. The use of Claim 1 wherein the monokine activity interfering agent inhibits the production of interleukin-1.

3. The use of Claim 1 wherein the monokine activity interfering agent inhibits the production of TNF.

4. The use of Claim 2 wherein the monokine activity interfering agent is a compound of the formula:

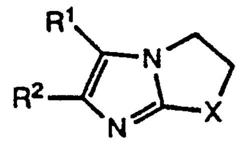

wherein:
One of $R^1$ and $R^2$ must be 4-pyridyl and the other is selected from monohalosubstituted phenyl wherein said substituent is selected from halo or $C_{1-4}$ alkoxy,
X is $CH_2$, $CH_2CH_2$ or S(0)n; and
n is 0, 1 or 2;
or a pharmaceutically acceptable salt thereof.

5 The use of Claim 4 wherein $R^1$ is 4-pyridyl; $R^2$ is 4-fluorophenyl; X is $S(O)_n$ and n is 0.

6. The use of Claim 4 wherein $R^1$ is 4-pyridyl; $R^2$ is 4-fluorophenyl; X is $S(O)_n$ and n is 1.

7. The use of Claim 4 wherein $R^1$ is 4-pyridyl; $R^2$ is 4-fluorophenyl; X is $S(O)_n$ and n is 2.

8. The use of Claim 4 wherein $R^1$ is 4-fluorophenyl; $R^2$ is 4-pyridyl; X is $S(O)_n$ and n is 0.

9. The use of Claim 4 wherein $R^1$ is 4-pyridyl; $R^2$ is 4-fluorophenyl; and X is $CH_2$.

10. The use of Claim 4 wherein $R^1$ is 4-pyridyl; $R^2$ is 4-methoxyphenyl; X is $S(O)_n$ and n is 0.

11. The use of Claim 4 wherein $R^1$ is 4-pyridyl; $R^2$ is 4-methoxyphenyl; and X is $CH_2$.

12. The use of Claim 4 wherein $R^1$ is 4-pyridyl; $R^2$ is 4-methoxyphenyl; and X is $CH_2CH_2$.

13. The use of Claim 2 wherein the monokine activity interfering agent is selected from a compound of the formula:

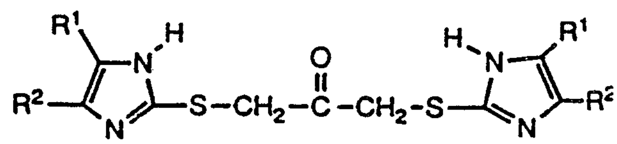

wherein:
one of $R^1$ and $R^2$ is 4-pyridyl and the other is monohalosubstituted phenyl;
or a pharmaceutically acceptable salt thereof.

14. The use of Claim 13 wherein $R^1$ is 4-pyridyl and $R^2$ is 4-fluorophenyl.

15. The use of Claim 2 wherein the monokine activity interfering agent is a compound of the formula:

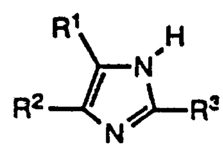

wherein:
One of $R^1$ and $R^2$ is 4-pyridyl and the other is selected from monohalosubstituted phenyl; and
$R^3$ is S or $SCF_2CF_2H$;
or a pharmaceutically acceptable salt thereof.

16. The use of Claim 15 wherein $R^2$ is 4-pyridyl, $R^1$ is 4-fluorophenyl and $R^3$ is either SH or $SCF_2SF_2H$.

17. The use of Claim 2 wherein the monokine activity interfering agent is selected from a compound of

the formula:

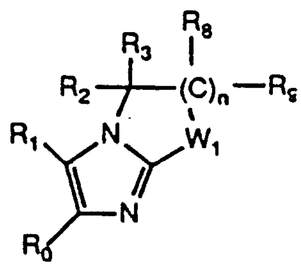

wherein:

$W_1$ is -($CR_4R_5$-($CR_6R_7$)-, -$CR_5$ = $CR_7$-, -N = $CR_7$-, -$S(O)_m$- or -O-;

one of $R_1$ and $R_0$ is 4-pyridyl or $C_{1-4}$ alkyl-4-pyridyl, provided that when $R_1$ is $C_{1-4}$ alkyl-4-pyridyl the alkyl substituent is located at the 2-position of the pyridine ring, and the other of $R_1$ and $R_0$ is

(a) phenyl or monosubstituted phenyl wherein said substituent is $C_{1-3}$ alkylthio, $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl, 1-acyloxy-1-alkylthio, $C_{1-2}$ alkoxy, halo, $C_{1-4}$ alkyl or Z wherein Z is - S-S - $Z_1$ and $Z_1$ is phenyl or $C_{1-9}$ alkyl; or

(b) disubstituted phenyl wherein said substitutents are, independently, $C_{1-3}$ alkylthio, $C_{1-2}$ alkoxy, halo or $C_{1-4}$ alkyl; or

(c) disubstituted phenyl wherein one of said substituents is $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl or 1-acyloxy-1-alkylthio and the other is $C_{1-2}$ aikoxy, halo, or $C_{1-4}$ alkyl; or

(d) disubstituted phenyl wherein the substituents are the same and are $C_{1-3}$ aikylsulfinyl, $C_{2-5}$ 1-alkenyl-1-thio, $C_{2-5}$ 1-alkenyl-1sulfinyl, $C_{3-5}$ 2-alkenyl-1-thio, $C_{3-5}$ 2-alkenyl-1-sulfinyl or 1-acyloxy-1-alkylthio or wherein the substituents together form a methylene dioxy group;

(e) monosubstituted phenyl wherein the substituent is

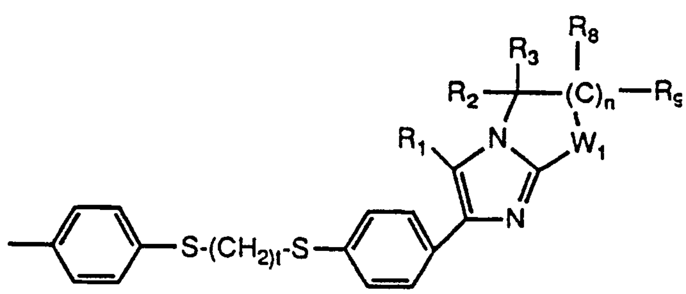

t is 0 or 1; $W_1$, $R_1$, $R_2$, $R_3$, $R_4$, $R_5$, $R_6$, $R_7$, $R_8$ and $R_9$ are as defined above;

provided that:

(1.) when $W_1$ is -($CR_4R_5$)-($CR_6R_7$)- then

n is 0 or 1; and

$R_2$, $R_3$, $R_4$, $R_5$, $R_6$, $R_7$, $R_8$, and $R_9$ are, independently, -H or $C_{1-2}$ alkyl; and

when $R_1$ or $R_0$ is 4-pyridyl, the other of $R_1$ and $R_0$ is other than mono-$C_{1-2}$ alkoxy-substituted phenyl or mono-halo-substituted phenyl; or

when n is 0, $R_4$ and $R_5$ together form an oxo; $R_4$ and $R_5$ are both fluoro, or one of $R_4$ and $R_5$ is H and the other OH; or

(2.) when $W_1$ is -$CR_5$ = $CR_7$- or -N = $CR_7$- then

n is 1;

$R_3$, $R_5$, $R_7$ and $R_9$ are, independently, -H or $C_{1-2}$ alkyl; and

$R_2$ and $R_8$ together represent a double bond in the B ring such that the B ring is an aromatic pyridine or pyrimidine ring;

(3.) when $W_1$ is $S(O)_m$ then

m is 0, 1 or 2:

n is 1 or 2; and

$R_3$ and $R_9$ are, independently, -H or $C_{1-2}$ alkyl;

$R_2$ and $R_8$ are, independently, -H or $C_{1-2}$ alkyl or $R_2$ and $R_8$ together represent a double bond in the B ring such that the B ring is an aromatic thiazole ring;
further provided that:
(a) when $R_2$ and $R_8$ are, independently, -H or $C_{1-2}$ alkyl and $R_1$ or $R_0$ is 4-pyridyl, then the other of $R_1$ and $R_0$ is other than mono-$C_{1-2}$ alkoxy-substituted phenyl or mono-halo-substituted phenyl; and
(b) when $R_2$ and $R_8$ together represent a double bond in the B ring such that the B ring is an aromatic thiazole ring, then m is 0 and n is 1; and
(4) when $W_1$ is -O- then
n is 1;
$R_3$ and $R_9$ are, independently, -H or $C_{1-2}$ alkyl; and
$R_2$ and $R_8$ together represent a double bond in the B ring such that the B ring is an aromatic oxazole ring;
or a pharmaceutically acceptable salt thereof.

18. The use of Claim 17 wherein:
$W_1$ is $-(CR_4R_5)-(CR_6R_7)-$, $-CR_5=CR_7-$, or $-S(O)_m-$;
one of $R_1$ and $R_0$ is 4-pyridyl or $C_{1-2}$ alkyl-4-pyridyl, provided that when $R_1$ is $C_{1-2}$ alkyl-4-pyridyl the alkyl substituent is located at the 2-position of the pyridine ring, and the other of $R_1$ and $R_0$ is
(a) monosubstituted phenyl wherein said substituent is $C_{1-2}$ alkylthio, $C_{1-2}$ alkylsulfinyl, 1-acyloxy-1-alkylthio, $C_{1-2}$ alkoxy or halo, or
(b) disubstituted phenyl wherein said substitutents are, independently, $C_{1-2}$ alkylthio or $C_{1-2}$ alkoxy, or
(c) disubstituted phenyl wherein one of said substituents is $C_{1-2}$ alkylsulfinyl or 1-acyloxy-1-alkylthio and the other is $C_{1-2}$ alkoxy, or
(d) disubstituted phenyl wherein the substituents are the same and are $C_{1-2}$ alkylsulfinyl or 1-acyloxy-1-alkylthio or wherein the substituents together form a methylene dioxy group;
provided that:
(1.) when $W_1$ is $-(CR_4R_5)-(CR_6R_7)-$ then
n is 0 or 1; and
$R_2$, $R_3$, $R_4$, $R_5$, $R_6$, $R_7$, $R_8$, and $R_9$ are -H; and
when $R_1$ or $R_0$ is 4-pyridyl, the other of $R_1$ and $R_0$ is other than mono-$C_{1-2}$ alkoxy-substituted phenyl or mono-halo-substituted phenyl;
(2.) when $W_1$ is $-CR_5=CR_7-$ then
n is 1;
$R_3$, $R_5$, $R_7$ and $R_9$ are -H; and
$R_2$ and $R_8$ together represent a double bond in the B ring such that the B ring is an aromatic pyridine ring;
(3.) when $W_1$ is $S(O)_m$ then
m is 0, 1 or 2;
n is 1 or 2; and
$R_3$ and $R_9$ are -H;
$R_2$ and $R_8$ are -H or $R_2$ and $R_8$ together represent a double bond in the B ring such that the B ring is an aromatic thiazole ring;
further provided that:
(a) when $R_2$ and $R_8$ are -H and $R_1$ or $R_0$ is 4-pyridyl, then the other of $R_1$ and $R_0$ is other than mono-$C_{1-2}$ alkoxy-substituted phenyl or mono-halo-substituted phenyl; and
(b) when $R_2$ and $R_8$ together represent a double bond in the B ring such that the B ring is an aromatic thiazole ring, then m is 0 and n is 1; and
(4) when $W_1$ is -O- then
n is 1;
$R_3$ and $R_9$ are -H; and
$R_2$ and $R_8$ together represent a double bond in the B ring such that the B ring is an aromatic oxazole ring;
or a pharmaceutically acceptable salt thereof.

19. The use of Claim 18 wherein the compound is:
2-(4-methylthiophenyl)-3-(4-pyridyl)-6,7-dinydro-[5H]-pyrrolo[1,2-a]imidazole;
2-(4-methylsulfinylphenyl)-3-(4-pyridyl)-6,7-dihydro-[5H]-pyrrolo[1,2-a]imidzole;
2-(4-ethylthiophenyl)-3-(4-pyridyl)-6,7-dihydro-[5H]-pyrrolo[1,2-a]imidazole;
2-(4-ethylsulfinylphenyl)-3-(4-pyridyl)-6,7-dihydro-[5H]-pyrrolo[1,2-a]imidazole;
2-(4-methylthiophenyl)-3-(4-(2-methyl)pyridyl)-6,7-dihydro-[5H]-pyrrolo[1,2-a]imidazole;
2-(4-methylsulfinylphenyl)-3-(4-(2-methyl)pyridyl)-6,7-dihydro-[5H]-pyrrolo[1,2-a]imidazole;
2-(4-methoxyphenyl)-3-(4-pyridyl)-imidazo[1,2-a]-pyridine;
5-(3,4-(methylenedioxy)phenyl-6-(4-pyridyl)-1,3-dihydroimidazo[2,1-b]thiazole;

2-(4-methoxyphenyl)-3-(4-(2-methyl)pyridyl)-6,7-dihydro-[5H]-pyrrolo[1,2-a]imidazole;
2-(4-acetoxymethylthiophenyl)-3-(4-(2-methyl)pyridyl)-6,7-dihydro-[5H]-pyrrolo[1,2-a]imidazole.
2-(trimethylacetylthiophenyl)-3-(4-pyridyl)--6,7-dihydro-[5H]-pyrrolo[1,2-a]imidazole;
6-(4-methylthiophenyl)-5-(4-pyridyl)-2,3-dihydro-imidazo-[1,2-b]thiazole;
5-(4-methylthiophenyl)-6-(4-pyridyl)-2,3-dihydro-imidazo[2,1-b]thiazole;
3-(4-methylthiophenyl)-2-(4-pyridyl)-6,7-dihydro-[5H]-pyrrolo[1,2-a]imidazole;
2-(4-propylthiophenyl)-3-(4-pyridyl)-6,7-dihydro-[5H]-pyrrolo[1,2-a]-imidazole;
2-(4-methylthiophenyl)-3-(4-(2-ethyl)pyridyl)-6,7-dihydro-[5H]-pyrrolo-[1,2-a]-imidazole;
2-(4-Mercaptophenyl)-3-(4-pyridyl)-6,7-hydro-[5H]-pyrrolo-[1,2-a]-imidazole disulfide;
2-(4-Methoxyphenyl)-3-(4-pyridyl)-7-oxo-5,6-dihydro-[7H]-pyrrolo[1,2-a]-imidazole;
5,6-dihydro-2-(4-Methoxyphenyl)-3-(4-pyridyl)-[7H]-pyrrolo-[1,2-a]-imidazole-7-ol; or
5,6-dihydro-7,7-difluoro-2-(4-Methoxyphenyl)-3-(4-pyridyl)-[7H]-pyrrolo-[1,2-a]-imidazole.

20. The use of Claim 3 wherein the monokine activity interfering agent is selected from a compound of Formula (V):

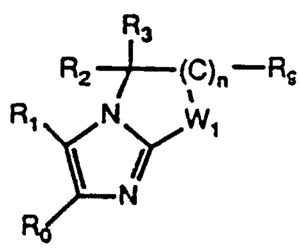

wherein:

$W_1$ is $-(CR_4R_5)-(CR_6R_7)-$, $-CR_5=CR_7-$, $-N=CR_7-$, $-S(O)_m-$ or -O-;

one of $R_1$ and $R_0$ is 4-pyridyl or $C_{1-4}$ alkyl-4-pyridyl, provided that when $R_1$ is $C_{1-4}$ alkyl-4-pyridyl the aikyl substituent is located at the 2-position of the pyridine ring, and the other of $R_1$ and $R_0$ is

(a) phenyl or monosubstituted phenyl wherein said substituent is $C_{1-3}$ alkylthio, $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl, 1-acyloxy-1-alkylthio, $C_{1-2}$ alkoxy, halo, $C_{1-4}$ alkyl or Z wherein Z is- S-S-$Z_1$ and $Z_1$ is phenyl or $C_{1-9}$ alkyl; or

(b) disubstituted phenyl wherein said substitutents are, independently, $C_{1-3}$ alkylthio, $C_{1-2}$ alkoxy, halo or $C_{1-4}$ alkyl; or

(c) disubstituted phenyl wherein one of said substituents is $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl or 1-acyloxy-1-alkylthio and the other is $C_{1-2}$ alkoxy, halo, or $C_{1-4}$ alkyl; or

(d) disubstituted phenyl wherein the substituents are the same and are $C_{1-3}$ alkylsulfinyl, $C_{2-5}$ 1-alkenyl-1-thio, $C_{2-5}$ 1-alkenyl-1-sulfinyl, $C_{3-5}$ 2-alkenyl-1-thio, $C_{3-5}$ 2-alkenyl-1-sulfinyl or 1- acyloxy-1-alkylthio or wherein the substituents together form a methylene dioxy group;

(e) monosubstituted phenyl wherein the substituent is

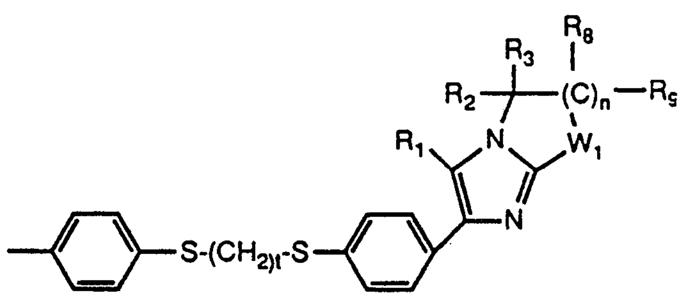

t is 0 or 1; $W_1$, $R_1$, $R_2$, $R_3$, $R_4$, $R_5$, $R_6$, $R_7$, $R_8$ and $R_9$ are as defined above;

provided that:

(1.) when $W_1$ is $-(CR_4R_5)-(CR_6R_7)-$ then n is 0; $R_4$ and $R_5$ may together form an oxo; $R_4$ and $R_5$ are both flouro; or one of $R_4$ and $R_5$ is H and the other OH; or

(2.) when $W_1$ is $-CR_5=CR_7-$ or $-N=CR_7-$ then

n is 1;

$R_3$, $R_5$, $R_7$ and $R_9$ are, independently, -H or $C_{1-2}$ alkyl; and
$R_2$ and $R_8$ together represent a double bond in the B ring such that the B ring is an aromatic pyridine or pyrimidine ring;
(3.) when $W_1$ is $S(O)_m$ then
m is 0, 1 or 2;
n is 1 or 2; and
$R_3$ and $R_9$ are, independently, -H or $C_{1-2}$ alkyl;
$R_2$ and $R_8$ are, independently, -H or $C_{1-2}$ alkyl or $R_2$ and $R_8$ together represent a double bond in the B ring such that the B ring is an aromatic thiazole ring;
further provided that:
(a) when $R_2$ and $R_8$ are, independently, -H or $C_{1-2}$ alkyl and $R_1$ or $R_0$ is 4-pyridyl, then the other of $R_1$ and $R_0$ is other a mono-fluoro-substituted phenyl; and
(b) when $R_2$ and $R_8$ together represent a double bond in the B ring such that the B ring is an aromatic thiazole ring, then m is 0 and n is 1; and
(4) when $W_1$ is -O- then
n is 1;
$R_3$ and $R_9$ are, independently, -H or $C_{1-2}$ alkyl; and
$R_2$ and $R_8$ together represent a double bond in the B ring such that the B ring is an aromatic oxazole ring;
or a pharmaceutically acceptable salt thereof.

21. The use of Claim 20 wherein
$W_1$ is $-(CR_4R_5)-(CR_6R_7)-$, $-CR_5=CR_7-$, or $-S(O)_m-$;
one of $R_1$ and $R_0$ is 4-pyridyl or $C_{1-2}$ alkyl-4-pyridyl,
provided that when $R_1$ is $C_{1-2}$ alkyl-4-pyridyl the alkyl substituent is located at the 2-position of the pyridine ring, and the other of $R_1$ and $R_0$ is
(a) monosubstituted phenyl wherein said substituent is $C_{1-2}$ alkylthio, $C_{1-2}$ alkylsulfinyl, 1-acyloxy-1-alkylthio, $C_{1-2}$ alkoxy or halo, or
(b) disubstituted phenyl wherein said substitutents are, independently, $C_{1-2}$ alkylthio or $C_{1-2}$ alkoxy, or
(c) disubstituted phenyl wherein one of said substituents is $C_{1-2}$ alkylsulfinyl or 1-acyloxy-1-alkylthio and the other is $C_{1-2}$ alkoxy, or
(d) disubstituted phenyl wherein the substituents are the same and are $C_{1-2}$ alkylsulfinyl or 1-acyloxy-1-alkylthio or wherein the substituents together form a methylene dioxy group;
provided that:
(1.) when $W_1$ is $-CR_5=CR_7-$ then
n is 1;
$R_3$, $R_5$, $R_7$ and $R_9$ are -H; and
$R_2$ and $R_8$ together represent a double bond in the B ring such that the B ring is an aromatic pyridine ring;
(2.) when $W_1$ is $S(O)_m$ then
m is 0, 1 or 2;
n is 1 or 2; and
$R_3$ and $R_9$ are -H;
$R_2$ and $R_8$ are -H or $R_2$ and $R_8$ together represent a double bond in the B ring such that the B ring is an aromatic thiazole ring;
further provided that:
(a) when $R_2$ and $R_8$ are -H and $R_1$ or $R_0$ is 4-pyridyl, then the other of $R_1$ and $R_0$ is other than mono-fluoro-substituted phenyl; and
(b) when $R_2$ and $R_8$ together represent a double bond in the B ring such that the B ring is an aromatic thiazole ring, then m is 0 and n is 1; and
(4) when $W_1$ is -O- then
n is 1;
$R_3$ and $R_9$ are -H; and
$R_2$ and $R_8$ together represent a double bond in the B ring such that the B ring is an aromatic oxazole ring;
or the pharmaceutically acceptab le salts thereof.

22. The use of Claim 20 wherein the compound is
2-Phenyl-3-pyridyl-6,7-dihydro-[5H]-pyrrolo-[1,2-a]imidazole;
2-4-Bromophenyl-3-pyridyl-6,7-dihydro-[5H]-pyrrolo-[1,2-a]-imidazole;
2-(4-Pyridyl)-3-(4-flurophenyl)-6,7-dihydro-[5H]-pyrrolo-[1,2-a] imidazole;
2-(4-Fluorophenyl)-3(4-pyridyl)-6,7-dihydro-[5H]-pyrrolo[1,2-a]imidazole;
2-(4-Methylthiophenyl)-3-(4-pyridyl)-6,7-dihydro-[5H]-pyrrolo[1,2-a]imidazole;

2-(4-Pyridyl)-3-(4-methylthiophenyl)-6,7-dihydro-[5H]-pyrrolo[1,2-a]imidazole;
2-(4-Methylthiophenyl)-3-[4-(2-methylpyridyl)]-6,7-dihydro-[5H]-pyrrolo[1,2-a]-imidazole;
2-(4-Methoxyphenyl)-3-(4-pyridyl)-7-oxo-5,6-dihydro-7H-pyrrolo[1,2-a]imidazole;
5,6-Dihydro-2-(4-methoxyphenyl)-3-(4-pyridinyl)-[7H]-pyrrolo[1,2-a]imidazole-7-ol;
2-(4-Acetoxymethylthiophenyl)-3-(4-pyridyl)-6,7-dihydro[5H]pyrrolo[1,2-a]-imidazole;
2(4-Methoxyphenyl)-3-(4-pyridyl)-6,7-dihydro-[5H]-pyrrolo[1,2-a]imidazole;
6-(4-Fluorophenyl)-5-(4′-pyridyl)-2,3-dihydroimidazo[2,1-b]thiazole;
6-(4-Fluorophenyl)-5-(4′-pyridyl)-2,3-dihydroimidazo[2,1-b]thiazole-1-oxide;
6-(4-Fluorophenyl)-5-(4-pyridyl)-2,3-dihydroimidazo[2,1-b]thiazole- 1,1-dioxide;
5-(4-Fluorophenyl)-6-(4′-pyridyl)-2,3-dihydroimidazo[2,1-b]thiazole;
2-(4-Methylsulfoxyphenyl)-3-(4-pyridyl)-6,7-dihydro[5H]-pyrrolo [1,2-a]imidazole;
2-(4-Ethylthiophenyl)-3-(4-pyridyl)-6,7-dihydro[5H]-pyrrolo-[1,2-a]imidazole; or
2-(4-Ethylsulfonylphenyl)-3-(4-pyridyl-6,7-dihydro-[5H]-pyrrolo-[1,2-a]imidazole.

23. The use of 5-(4-pyridyl)-6(4-fluorophenyl)-2,3-dihydroimidazo-(2,1-b)-thiazole in the manufacture of a medicament for treating HIV infection in a human afflicted with human immunodeficiency virus (HIV) infection.

24. The use of 2-(4-methylthiophenyl)-3-(4-pyridyl)-6,7-dihydro-[5H]-pyrrolo[1,2-a]-imidazole in the manufacture of a medicament for treating HIV infection in a human afflicted with human immunodeficiency virus (HIV) infection.

25. The use of 2-(4-Ethylthiophenyl)-3-(4-pyridyl)-6,7-dihydro-[5H]-pyrrolo-[1,2-a]-imidazole in the manufacture of a medicament for treating HIV infection in a human afflicted with human immunodeficiency virus (HIV) infection.

26. The use of 2-(4-Methylthiophenyl)-3-[4-(2-methylpyridyl)]-6,7-dihydro-[5H]-pyrrolo-[1,2-a]-imidazole in the manufacture of a medicament for treating HIV infection in a human afflicted with human immunodeficiency virus (HIV) infection.

27. The use of 6-(4-Fluorophenyl)-5-(4-pyridyl)-2,3-dihydroimidazo[2,1-b]thiazole-1,1-dioxide in the manufacture of a medicament for treating HIV infection in a human afflicted with human immunodeficiency virus (HIV) infection.

Figure 1

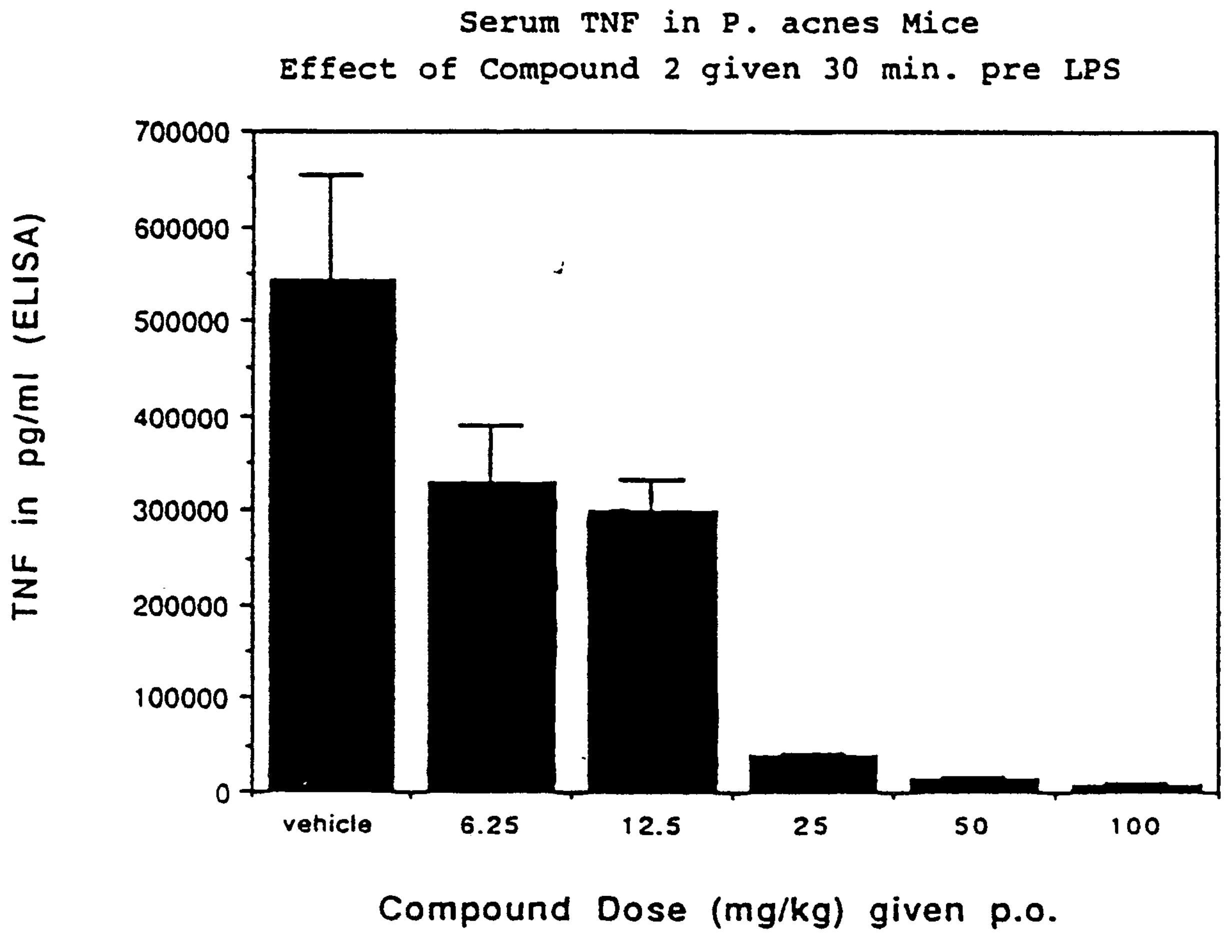
Serum TNF in P. acnes Mice
Effect of Compound 2 given 30 min. pre LPS
TNF in pg/ml (ELISA)
700000
600000
500000
400000
300000
200000
100000
0
vehicle
6.25
12.5
25
50
100
Compound Dose (mg/kg) given p.o.

Figure 2

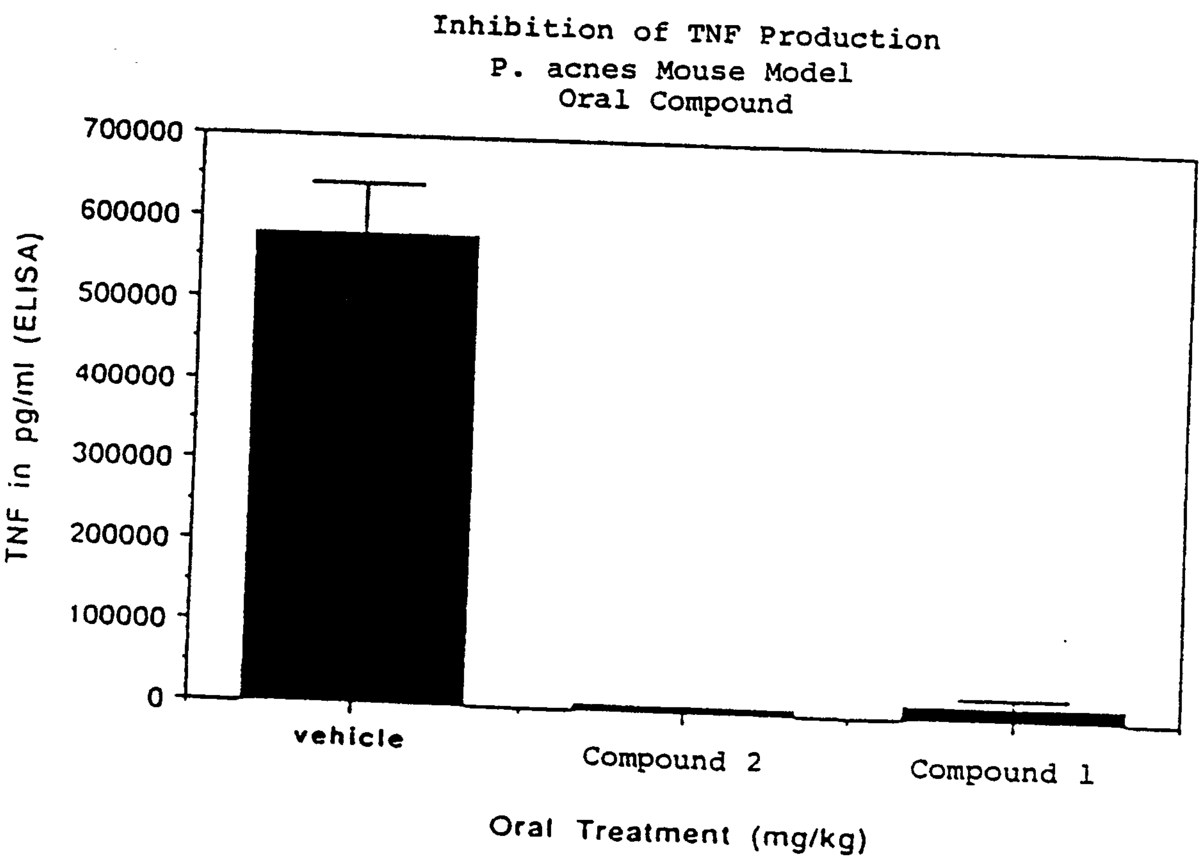
Inhibition of TNF Production
P. acnes Mouse Model
Oral Compound
TNF in pg/ml (ELISA)
700000
600000
500000
400000
300000
200000
100000
0
vehicle
Compound 2
Compound 1
Oral Treatment (mg/kg)

Figure 3

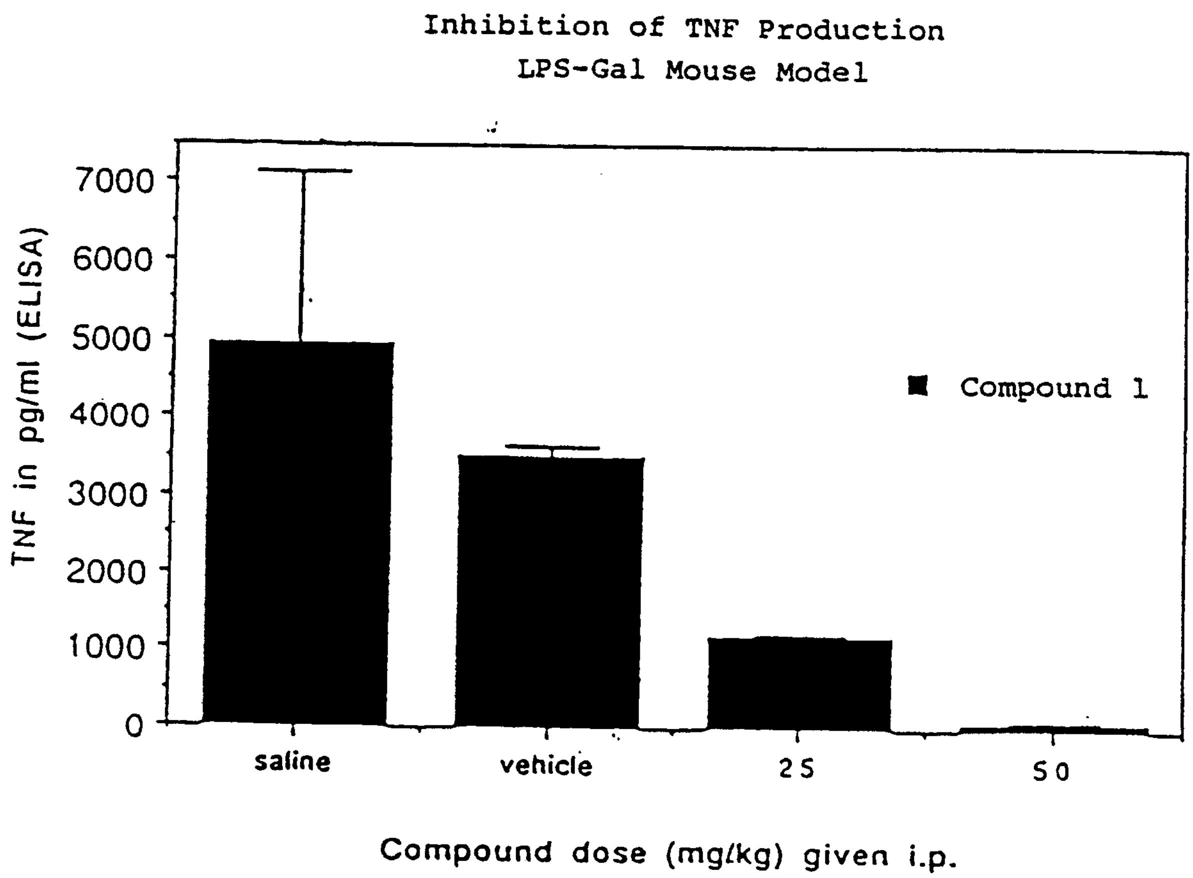
Inhibition of TNF Production
LPS-Gal Mouse Model
7000
6000
5000
4000
3000
2000
1000
0
TNF in pg/ml (ELISA)
Compound 1
saline
vehicle
25
50
Compound dose (mg/kg) given i.p.

Figure 4

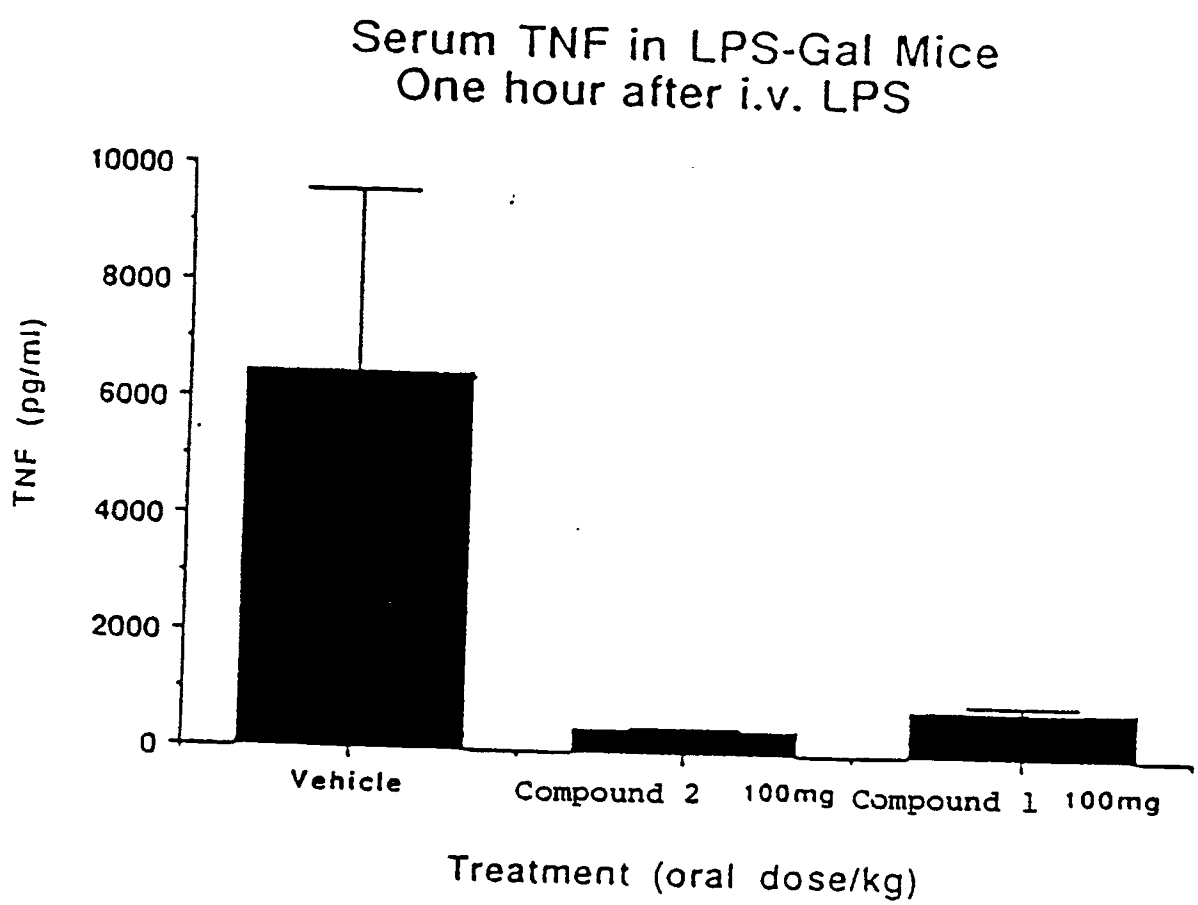
Serum TNF in LPS-Gal Mice
One hour after i.v. LPS
10000
8000
6000
4000
2000
0
TNF (pg/ml)
Vehicle
Compound 2 100mg
Compound 1 100mg
Treatment (oral dose/kg)

Figure 5

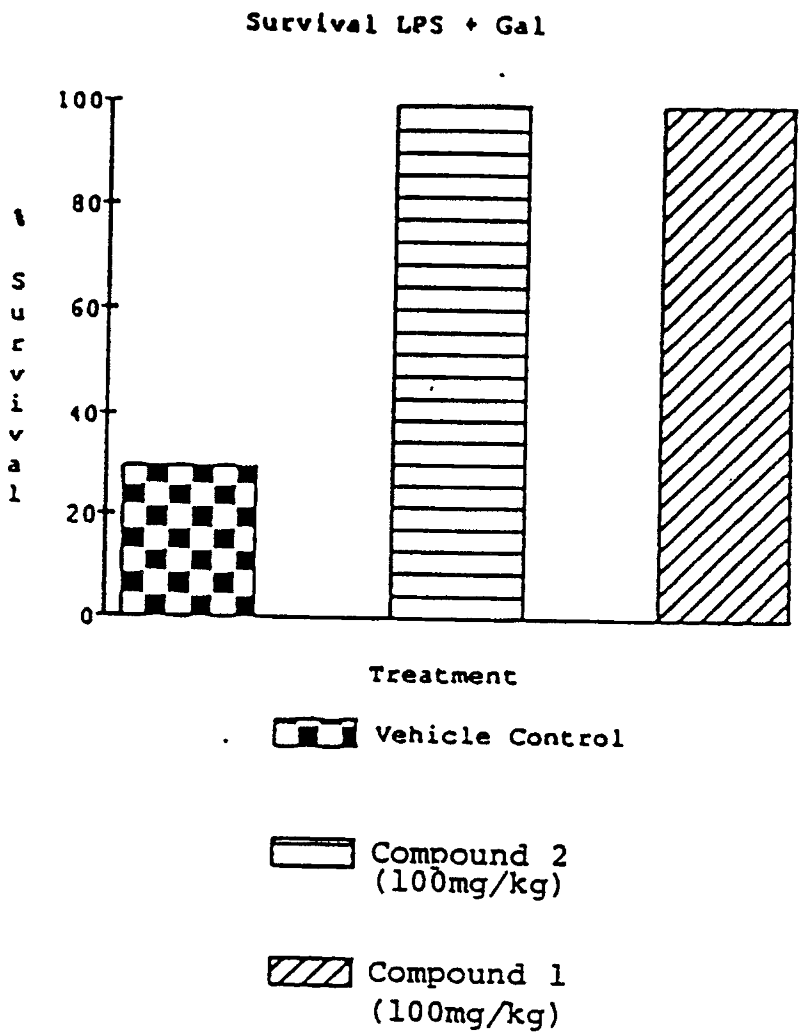
Survival LPS + Gal
% Survival
100
80
60
40
20
0
Treatment
Vehicle Control
Compound 2 (100mg/kg)
Compound 1 (100mg/kg)